# EUROPEAN PATENT SPECIFICATION

(11) **EP 3 334 405 B1**
(45) Date of publication and mention of the grant of the patent: **29.09.2021**
(21) Application number: 16757408.6
(22) Date of filing: 10.08.2016
(51) Int. Cl.: A61K 8/41, A61Q 5/00, A61K 8/60, A61Q 15/00, A61Q 17/00, A61Q 19/00, A61K 8/86, A61K 8/06, A61K 8/11, A61K 8/14, A61K 8/73

(54) **SKIN PENETRATING DELIVERY SYSTEM BASED ON POSITIVELY CHARGED SUGAR EMULSIFIERS**
HAUT PENETRIERENDES TRÄGERSYSTEM AUF BASIS VON POSITIV GELADENEN ZUCKERTENSIDEN.
SYSTÈME VECTEUR PERMETTANT LE PASSAGE DANS LA PEAU BASÉ SUR DES TENSIO ACTIFS À BASE DE SUCRES POSITIVEMENT CHARGÉS.

(30) Priority: 10.08.2015 US 201562203142 P
(43) Date of publication of application: 20.06.2018
(73) Proprietor: Evonik Operations GmbH, 45128 Essen (DE)
(72) Inventor: MUNK, Christian, 36396 Steinau-Marborn (DE); SACHER, Michael Manfred, 36381 Schluchtern-Breitenbach (DE); BEYER, Monika, 61130 Nidderau-Ostheim (DE)
(74) Representative: Evonik Patent Association
(86) International application number: PCT/US2016/046313
(87) International publication number: WO 2017/027570

(56) References cited:
- EP-A2- 2 340 805
- US-A1- 2011 302 725
- US-A1- 2014 134 221
- DATABASE GNPD [Online] MINTEL; May 2013 (2013-05), "Cream Soda Smoothing Cream", XP002762783, Database accession no. 2038767
- DATABASE GNPD [Online] MINTEL; April 2014 (2014-04), "Corrector Concentrate Dark Spots", XP002762784, Database accession no. 2314212
- Personal Care Products Council: "International Cosmetic Ingredient Dictionary and Handbook-Fourteenth Edition-Introduction INCI Name Monographs A-H", 2012, T.E. Gottschalck, J.E. Bailey, Washington, D.C., XP002762785, ISBN: 1-882621-48-4 vol. 1, pages 316-316, Behentrimonium chloride; page 316 - page 316
- "Effects of Selected Polysorbate and Sucrose Ester Emulsifiers on the Physicochemical Properties of Astaxanthin Nanodispersions", MOLECULES, vol. 18, 2013, - 2013, pages 768-777, XP002762786, ISSN: 1420-3049

## Description

### BACKGROUND OF THE INVENTION

The present invention relates generally to the field of cosmetic actives and delivery systems and more particularly to compositions comprising a positively charged water based penetrating sugar based delivery system with the ability to encapsulate at least one or a combination of oil soluble/oil miscible/oil dispersible cosmetic actives and their use in personal care compositions. The inventive compositions are capable of penetrating the skin thereby delivering the cosmetic actives into the skin.

It is known, that delivery systems based on hydrophobized polysaccharides do not penetrate the skin or hair and show good film forming properties;
- U.S. 2014/0134221 A1 *Agent for providing positive surface charge on UV Protection Vesicles*
- U.S. 2011/0200666 A1 *UV Protection Agent for the skin or hair having a UV filter substance encapsulated in a vesicular carrier system*
- U.S. 8,152,860 B2 *Cosmetic composition for coloring hair comprising a direct hair dye and a carrier system comprising polysaccharides*
- IP.Com - IPCOM000216981D *Sun Protection Agents and Uses Thereof*
- IP.Com - IPCOM000221091D *Penetrating and Non Penetrating Carrier Systems Including Cosmetic Active Ingredients and Uses Thereof* which disclosesa combination of a non penetrating carrier system based on hydrophobized polysaccharides and a penetrating carrier system based on ceramides, phospholipids, glycosphingolipids and/or diacylglycosides.
- DE 102009044891 A1, EP 2340805A2 Holder system for enclosing lipophilic agents and oil in high concentrations (G. Blume)

### BRIEF SUMMARY OF THE INVENTION

The present invention comprises a physically stable, positively charged water based composition consisting of a sugar based delivery system and at least one lipophilic/oil miscible/oil dispersible active ingredient encapsulated to be used in Personal Care products including Cosmetics.

The following definitions are provided and used throughout this disclosure:
- "Composition" means and comprises a positively charged sugar based vesicle made of at least a sugar based emulsifier, coemulsifiers, positively charged molecules, solvents, retinol as lipophilic active ingredient and optional auxiliary materials like chelating agents and preservatives. At least one coemulsifier comprises a polyoxyethylene (polysorbate) molecule.
- "Sugar based delivery system" means and comprises at least a sugar based emulsifier, coemulsifiers and positively charged molecules,
- "Lipophilic active ingredient" means an oil soluble/oil miscible/oil dispersible substance chosen from e.g. vitamins, antioxidants, plant extracts. synthetic molecules with e.g. anti aging, skin soothing, skin tone, anti cellulite. anti inflammatory, whitening, improoving overall skin condition properties. Lipophilic active ingredients are characterized as having a log pOW > about 0, typically greater than about 1,greater than about 3 and in some cases greater than about 5 to greater than about 6. The lipophilic active ingredient comprises at least retinol.
- "Personal Care" means and comprises any toiletry and topical care products including. without limitation, leave-on personal care products (i.e., products that are left on keratinous substrates after application); rinse-off personal care products (i.e., products that are washed or rinsed from keratinous substrates during or within a few minutes of application); at least one of shampoos; chemical and non-chemical hair curling and hair straightening products; hair style maintaining and hair conditioning products; lotions and creams for nails, hands. feet, face, scalp and/or body; hair dye; face and body makeup; nail care products; astringents; deodorants; antiperspirants; antiacne; antiaging; depilatories; colognes and perfumes; skin protective creams and lotions (such as sunscreens): skin and body cleansers; skin conditioners; skin toners; skin firming compositions; skin tanning and lightening compositions; liquid soaps; bar soaps; bath products; shaving products; and oral hygiene products (such as toothpastes, oral suspensions, and mouth care products). The form of such Personal Care products is not limited and may be without limitation, a liquid, gel, spray, emulsion (such as lotions and creams), shampoo, pomade, foam, tablet, stick (such as lip care products), makeup, suppositories, among others, any of which can be applied to the skin or hair and which typically are designed to remain in contact there with until removed, such as by rinsing with water or washing with shampoo or soap. Other forms could be gels that can be soft, stiff, or squeezable. Emulsions can be oil-in-water, water-in-oil, water-and-siiicone, or multiphase. Sprays can be non-pressurized aerosols delivered from manually pumped finger-actuated sprayers or can be pressurized aerosols such as mousse, spray, or foam forming formulation, where a chemical or gaseous propellant is used. "Cosmetic" means and comprises a Personal Care product comprising a powder, lotion, lipstick, rouge, or other preparation for affecting the appearance of or beautifying the face, skin, hair, nails or other keratinous materials.
- "Physically stable" means, that a given composition had not formed two visually distinct and separated layers after being stored at elevated temperature of 40°C for 30 days.
- "Penetrating" or "Skin Penetrating" means penetration into the skin proven by 5D Intravitaltomography as described by Schwarz et al. A comparative study of different instrumental concept for spectrally and lifetime-resolved multiphoton Intravital tomography (5D-IVT) in dermatological applications. Proc. of SPIE Vol.7568, 75680D-1*:* Multiphoton optical tomography or intravital tomography (IVT) provides non-invasive optical sectioning of biological specimens such as skin. Additional information beyond morphology can be obtained by analyzing the collected fluorescence light spectroscopically and by means of its fluorescence decay time. This is frequently termed spectral fluorescence lifetime imaging (SFLlM) or 5D-intravital tomography (5D-IVT). Spectral and temporal resolution scales with the number of detection increments (i.e. spectral channels and time bins). 5D-IVT enables us to detect new physiological parameters, however accompanied by a decrease in intensity per channel

In one aspect of the invention, the inventive delivery system has stabilizing effects on instable active ingredients as well as reduces the skin irritation potential of active ingredients (e,g., as confirmed by 5D Intravitaltomography). Stability is visually determined after exposing a delivery system to a temperature of 40C for a period of 30 days. A stable or stabilized mixture is one without layers or phase separation.

The various aspects of the invention can be used alone or in combinations with each other.

### BRIEF DESCRIPTION OF THE DRAWINGS

Figure 1 is a graphical representation of a Delivery System In accordance with this invention.
Figure 2 is a graphical representation of the stability of retinol encapsulated by the inventive delivery system.
Figure 3 is a photomicrograph showing penetration of active ingredients into skin stratum.
Figure 4 is a graphica representation of skin irritation for the active ingredients shown in Figure 3.

### DETAILED DESCRIPTION OF THE INVENTION

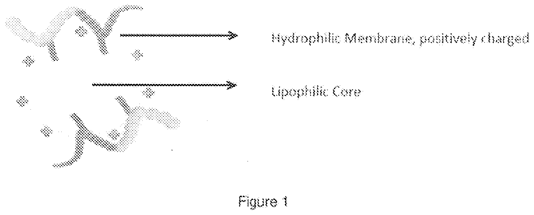

Broadly, the present invention relates to a physically stable and skin penetrating composition of positively charged Sugar based Delivery System (oil in water or o/w system) that encapsulates at least one oil soluble cosmetic active compound, and methods for making and using the encapsulated cosmetic active compound. The Delivery System is dispersed into an aqueous phase wherein the aqueous phase comprises about 10 to about 80wt.% of the composition, in some cases about 30 to about 80 wt.%, in some cases about 40 to about 80wt.% and in some cases about 40 to about 50wt.%. Referring now to Figure 1, Figure 1 illustrates a composition of the invention wherein a lipophilic core comprising an oil soluble cosmetic active compound is encapsulated by a sugar based delivery system. The composition can be characterized as comprising an oil in water emulsion wherein the sugar backbone of delivery system extend into the hydrophilic membrane and theail portions (e.g., alkyl tail portions), of the delivery system extend into the lipophilic portion.

The Delivery System comprises a sugar based emulsifier. a minimum of one coemulsifier or a combination of coemulsifiers chosen from coemulsifiers 1, 2 or 3, a positively charged molecule, solvent, a lipophilic/oil miscible/oil dispersible active ingredient as defined above and optional auxiliary material like chelating agents and/or preservatives and/or gelling and stabilizing agents.

As described below in greater detail, the lipophilic components are combined and the hydrophilic components are combined in the water phase and then the combinations are blended and homogenized under high pressure in order to form the inventive composition. The membrane comprises at least one sugar based emulsifier, at least one coemulsifier and a positively charged molecule.

The sugar based emulsifier comprises a polysugar backbone selected from a polyfructose based inulin modified by linear C8-C22 alkyl groups.

Molecular weight of the sugar based emulsifier is 5.000 - 500.000 g/mol, preferably 5.000 - 100.000 g/mol.
Percentage of attached alkyl groups or degree of modification is 0.01 - 0.09, preferably 0.03 - 0,15.

In some cases, it is also possible to use mixtures of the aforementioned polysaccharides.
Typical amounts of the sugar based emulsifier are about 1 to about 20 wt% of the composition, and preferably about 1 to about 5 wt% of the composition.

### Coemulsifier 1

The optional sucrose based coemulsifier can be selected from a wide range of suitable modified sucrose molecules. It is preferably modified by linear or branched alkyl groups, preferably linear alkly groups from C8-C22. Preferred molecules are α-d-Glucopyranoside, β-d-fructofuranosyl, dodecanoate and α -d-Glucopyranoside, β-d-fructofuranosyl, mixed palmilates and stearates and α-d-Glucopyranoside, β-d-fructofuranosyl, monoteteradecanoate and α-d-Glucopyranoside, β-d-fructofuranosyl, mono[(Z)-9-octadecenoate] and α-d-Glucopyranoside, β-d-fructofuranosyl, didodecanoate and α -d-Glucopyranoside, β-d-fructofuranosyl, mixed dipalmitates and distearates and α-d-Glucopyranoside, β-d-fructofuranosyl, diteteradecanoate and α-d-Glucopyranoside, β-d-fructofuranosyl, di[(Z}-9-octadecenoate].
In special compositions it is also possible to use mixtures of the aforementioned sucrose based coemulsifiers.

Typical amounts of Coemulsifier 1 are about 0,01 to about 5 wt%, and preferably about 0,05 to about 0,5 wt% of the composition.

### Coemulsiffer 2

The Coemulsifier 2 is a polyoxyethylene based coemulsifier comprising polysorbate molecule can be selected from a wide range of suitable molecules. It is consisting of 4-1000 oxyethylene groups esterified with fatty acids, It is preferably consisting of 4-100 oxyethylene groups esterified with 1-5 fatty acids (C8-C22), most preferably 20 oxyethylene groups esterified with 1 fatty acid (C8-C22). In special compositions it is also possible to use mixtures of the aforementioned polyoxyethyfene based coemulsifiers. Typical amounts of Coemulsifier 2 are 0.1-20 wt%, preferably 0,5-10 wt%. Without wishing to be bound by an theory of explanation, it is believed that the coemulsifier enhances the penetration of active ingredients into the skin. Such activie ingredient penetration is a marked improvement over conventional delivery systems and is suprising and an unexpected result. Coemulsifier 3

The optional Coemulsifier 3 can be selected from a wide range of available emulsifiers: In products in which emulsifiers are employed, any suitable cosmetic emulsifier having a hydrophilic-lipophilic balance (HLB) in the range of about 1 to about 20 can be used The emulsifier can be nonionic, cationic, anionic, or amphoteric or a combination of such emulsifiers can be used.

Examples of nonionic emulsifiers are at least one of laureths, e.g. laureth-4; ceteths, e.g, ceteths-1; polyethylene glycol cetyl ether: ceteareths, e.g. ceteareth-25; polyglycol fatty acid glycerides; hydroxylated lecithin; lactyl esters of fatty acids; and alkyl polyglycosides.

Examples of cationic emulsifiers are at least one of cetyldimethyl-2-hydroxyethylammonium dihydrogenphosphate; cetyltrimonium chloride; cetyltrimonium bromide; cocotrimonium methosulfate; as well as emulsifiers that contain a quaternized nitrogen.

Anionic emulsifiers include, for example, at least one of alkyl sulfates; alkyl ether sulfates; alkylsulfonates; alkylarylsulfonates; alkyl succinates; alkyl sulfosuccinates: N-alkylsarcosinates; acyl taurates; acyl isethionates; alkyl phosphates; alkyl ether phosphates; alkyl ether carboxylates; alpha-olefinsulfonates, and the alkali metal and alkaline earth metal salts of such materials (e.g, sodium, potassium, magnesium, calcium) as well as ammonium. trialkylamine. trialkanol amine, and alkylalkanol amine salts. The alkyl ether sulfates, alkyl ether phosphates and alkyl ether carboxylates may include ethylene oxide or propylene oxide units.

Typical amounts of Coemulsifier 3 are about 0,1 to about 20 wt%, and preferably about 0,5 to about 10 wt% of the composition.

### Positively charged molecule

The inventive composition comprises at least one positively charged molecule. The positively charged molecule is associated with membrane. The positively charged molecules can have a zeta potential of about 1 mV to about 150mV. about 10mV to about 100mV and in some cases about 20mV to about 50mV (as measured by zeta potential by means of laser Doppler electrophoresis as given later in the description). The optional positively charged molecule can be selected from one or more of the following charge donors a) to f):
a) N-Stearyl-N,N-dimethyl-N-(2-hydroxy-3-panthenyl)propylammonium Salts with the Formula (I) 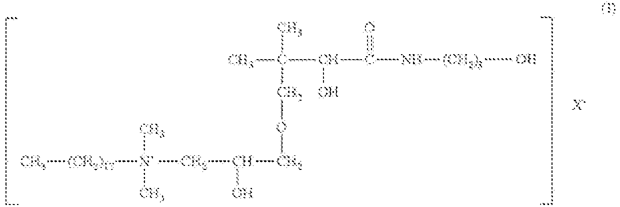 ∘ in which X⁻ in the aforementioned formula (I) is a cosmetically or pharmaceutically compatible organic or inorganic anion. X⁻ is preferably a halide ion or the anion of an organic acid chosen among a cosmetically or pharmaceutically compatible carboxylic acid or sulfonic acid. With particular preference X⁻ is bromide, chloride, fluoride, iodide, saccharinate, tosylate or methosulfate. with particular preference chloride.
b) Positively Charged Quaternary Sugar Derivatives (Sugar Quats) with the Formula (II) 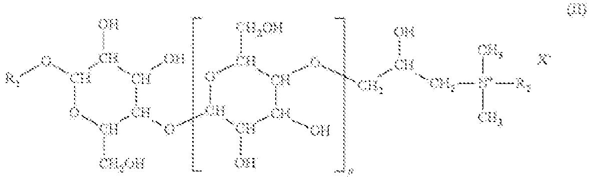
   - The quaternary sugar derivatives (sugar quats) preferably have monosaccharide units with 6 carbon atoms. The sugar quats with particular preference are chosen among glucose, fructose, mannose and/or galactose. In particularly preferred variants monosaccharides [form] disaccharide units. Sugar quats in which the monosaccharides form the disaccharide units maltose and/or lactose are particularly preferred.
      - The quaternary sugar derivatives are preferably ether compounds of alkyl glucopyranosides (n=0) and/or alkyl glucopyranosylglucopyranosides (n-1) with N-alkyl-N,N-dimethyl-N-{2,3-dihydroxypropyl}ammonium salts and their combinations. The alkyl group R₁ bonded to the sugar unit then preferably has an unbranched or branched alkyl chain with C₄-C₁₄, especially C₄, C₁₀ and/or C₁₂ carbon atoms. The alkyl group R₂ bonded to the quaternary nitrogen atom is preferably a methyl group or with particular preference as an unbranched or branched alkyl chain with C₄-C₁₈ and especially C₄, C₁₂ and/or C₁₈ carbon atoms.
      - The aforementioned ether compounds can be uniform substances. However, mostly natural oils are generally used to produce these substances, preferably coconut oil and palm oil/palm kernel oil, so that in addition to the primarily mentioned unbranched or branched alkyl groups R₁ and R₂, additional alkyl and alkenyl groups of other chain lengths can be present in subordinate amounts in the substance mixture according to the chosen starting material.
      - The quaternary sugar derivatives with formula (II) also explicitly include oligosaccharide compounds in which the number of monosaccharides units n is preferably 1<n<100. R₁ preferably represents a hydrogen atom and R₂ preferably an alkyl group with C₃-C₂₀ and especially C₁₈ carbon atoms.
      - The anion X⁻ in the aforementioned formula (II) is preferably a halide ion or the anion of an organic acid chosen among cosmetically or pharmaceutically compatible carboxylic acid or sulfonic add. With particular preference X⁻ is bromide, chloride, fluoride, iodide, saccharinate, tosylate or methosutfate, with particular preference chloride.
c) Positively Charged Phospholipids Consisting of Diester and Triester Phosphatides with the Formula (III) 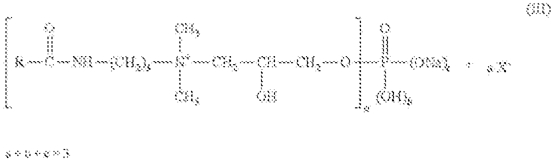
   - The positively charged phospholipids and their salts with formula (III) preferably have an unbranched or branched alkyl group and/or alkenyl group R with C₇-C₂₅ and especially C₁₃-C₂₁ carbon atoms. With particular preference the alkyl group R has C₁₃, C₁₇ and/or G₂₁ carbon atoms. In the case of an alkenyl group R with particular preference has chains with C₁₇ carbon atoms. One, two or three double bonds then preferably He in the alkenyl group.
   - Mostly natural oils are used to produce these substances, preferably coconut oil, palm oil/palm kernel oil, sunflower oil, thistle oil, olive oil, grapeseed oil, borage oil and castor oil, etc. so that in addition to the primarily mentioned unbranched or branched alkyl and/or alkenyl groups R, additional alkenyl and alkyl groups of other chain lengths can be present in subordinate amounts of the substance mixture according to the chosen starting material. In the case of castor oil the C₁₇ alkenyl group has a functional hydroxyl group.
   - The anion X⁻ in the aforementioned formula is preferably a halide ion or the anion of an organic acid chosen among cosmetically or pharmaceutically compatible carboxylic acid or sulfonic acid. With particular preference X⁻ is bromide, chloride, fluoride, iodide, saccharinate. tosylate or methosulfate. with particular preference chloride.
d) Positively Charged N-(3-alkylamido)propyl-N,N-dimethyl-N-(2,3-dihydroxypropyl)-Ammonium Salts or N-(3-alkenylamido)propyl-N,N-dimethyl-N-(2,3-dihydroxypropyl)-Ammonium Salts with Formula (IV) 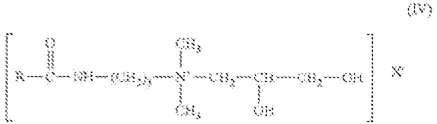
   - or N-(3-ricinylamido)propyl-N,N-dimethyl-N-(2,3-dihydroxypropyl)ammonium salts.
   - The positively charged N-(3-alkylamido)propyl-N,N-dimethyl-N-(2,3-dihydroxypropyl)-ammonium salts of formula (IV) preferably have an unbranched or branched alkyl group R with C₇-C₂₅ and especially C₁₃-C₂₁ carbon atoms. With particular preference the alkyl group hes C₁₃, C₁₇ and/or C₂₁ carbon atoms. The N-(3-alkylamido)propyl-N,N-dimelhyl--N-(2.3-dihydroxypropyl)ammonium salts with formula (IV) can be uniform substances, but generally natural oil are mostly used to produce these substances, preferably coconut oil and palm oil/palm kernel oil, but optionally also tallow so that, *in* addition to the primarily mentioned unbranched or branched alkyl groups R, additional alkyl and alkenyl groups of other chain lengths can be present in subordinate amounts in the substance mixture according to the chosen starting material.
   - The anion X⁻ in the aforementioned formula is preferably a halide ion or the anion of an organic acid chosen among cosmetically or pharmaceutically compatible carboxylic acid or sulfonic acid. With particular preference X⁻ is bromide, chloride, fluoride, Iodide, saccharinate, tosylate or methosulfate, with particular preference chloride.
   - The N-(3-alkenylamido)propyl-N,N-dimethyl-N-(2,3-dihydroxypropyl)ammonium salts with formula (IV) preferably have an unbranched or branched alkenyl group R with C₁₃-C₂₃ and especially C₁₇ carbon atoms. One, two or three double bonds are then preferably present in the alkenyl group.
   - The N-(3-alkenylamido)propyl-N,N-dimelhyl-N-(2,3-dihydroxypropyl)ammonium salts with formula (IV) can be uniform substances but generally natural oils are mostly used to produce these substances, preferably sunflower oil, thistle oil, olive oil, grapeseed oil, borage oil and castor oil, etc. so that, in addition to the primarily mentioned unbranched or branched alkenyl groups R, additional alkenyl and alkyl groups of other chain lengths can be present in subordinate amounts in the substance mixture according to the chosen starting material
   - The anion X⁻ in the aforementioned formula is preferably a halide ion or the anion of an organic acid chosen among cosmetically or pharmaceutical compatible carbolic acid or sulfonic acid. With particular preference X⁻ is bromide, chloride, fluoride, iodide, saccharinate, tosylate or methosulfate, with particular preference chloride.
e) Positively Charged Quaternary N.N,N-trimethyl-N-(2-hydroxy-3-"R ether"-propyl)-Ammonium Salts With Formula (V) 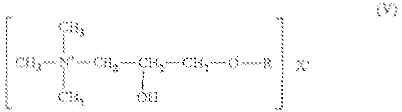
   - The quaternary N,N,N-trimethyl-N-(2-hydmxy-3-"R ether"-propyl)ammonium salts with formula (V) preferably have a group R which originates from natural sources. R with particular preference represents honey, hyaluronic acid, the polysaccharides xanthan gum and trehalose as well as hydrotyzates of silk, wheat starch, corn starch, keratin and protein hydrolyzates from wheat, rice, soya, oats, etc.
   - The anion X⁻ in the aforementioned formula is preferably a halide ion or the anion of an organic acid chosen among cosmetically or pharmaceutically compatible carboxylic acid or sulfonic acid. With particular preference X⁻ is bromide, chloride, fluoride, iodide, saccharinate, tosylate or methosulfate.
   - As an alternative the vesicles, in addition to the charge donors mentioned above, can have one or more of the following additional charge donors:
f) Positively Charged Quaternary C₁₂-C₂₂ Alkyltrimethylemmoinium Salts (or Fatty Acid Trimonium Salts) With Formula (VI) 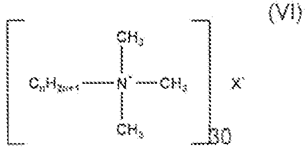
   - The quaternary alkyltrimethylammonium salts (or fatty acid trimonium salts) with formula (VI) preferably an alkyl group with C₁₂-C₂₂ carbon atoms. n in the aforementioned alkyltrimethylammonium salt formula is preferably equal to 22.
   - X⁻ in the aforementioned formula (VI) is a halide ion or the anion of an organic acid chosen among cosmetically or pharmaceutically compatible carboxylic acid or sulfonic acid. With particular preference X⁻ is bromide, chloride, fluoride, iodide, saccharinate, tosylate or methosulfate.

Typical amounts of the positively charged molecule are about 0,1 to about 10 wt%, and preferably, about 0,5 to about 2 wt% of the composition. While any suitable charged molecule can be used. desirable results can be obtained by using member a) and/or f) above. Oil

In one aspect of the invention, at least one oil/emollient might be included in the composition being part of the inner core of the vesicle. Oil/emollients and solublizing agents can be comprising of volatile oils and/or nonvolatile oils. Nonvolatile oils mean those which remains on the surface of the skin at ambient temperature 25°C. These nonvolatile oils can be hydrocarbon oils, in particularly animal or vegetable origin, silicone oils or their mixtures. The term Hydrocarbon oil means an oil comprising hydrogen and carbon atoms and optionally oxygen, nitrogen sulphur and/or phosphorus atoms. The nonvolatile oils can in particularly be chosen from nonvolatile hydrocarbon oils, if appropriate fluorinated, and/or nonvolatile silicone oils. Hydrocarbon oils origin from animal, plants or synthetic, such as triglycerides composed of fatty acids and of glycerol.

The fatty acid can be varied chain length from C4-C24. These chains can be saturated or unsaturated, and linear or branched. These oils can be triglyceride of heptanoic acid or octanoic acid, perhydrosqualene; hydrocarbon-based plant oils such as liquid triglycerides of fatty acids ranging from 4 to 24 carbon atoms, for instance heptanoic or octatonic acid triglyceride, or alternatively sunflower oil, maize oil, soybean oil, marrow oil grapeseed oil, sesame seed oil, hazelnut oil, apricot oil, macadamia oil, castor oil, avocado oil, caprylic/capric acid triglycerides, jojoba oil and shea butter; linear or branched hydrocarbons of mineral or synthetic origin, such as liquid paraffin and derivatives thereof, petroleum jelly, polydecenes, polybutenes and hydrogenated polyisobutene, such as parleam: synthetic esters and ethers, such as of fatty acids, for instance the oils of formula R,COOR- wherein R, is chosen from higher fatty acid residues comprising from 1 to 40 carbon atoms and R∼ is chosen from hydrocarbon-based chains comprising from 1 to 40 carbon atoms, wherein purcellin oil, isononyl isononanoate, isopropyl myristate, 2-ethylhexyl palmitate, 2-octyldodecyl stearate, 2-octyldodecyl erucate, isostearyl isostearate or tridecyl trimellitate; hydroxylated esters. for instance isostearyl lactate, octyl hydroxystearate, octyldodecyf hydroxystearate, diisostearyl malate, triisocetyl citrate and fatty alkyl heptanoates, octanoates or decanoates; polyol esters. for instance propylene glycol dioctanoate, neopentyl glycol diheptanoate or diethylene glycol diisononanoate; and pentaerythritol esters. for instance pentaerythrityl tetraisostearate; fatty alcohols comprising from 12 to 26 carbon atoms, for instance oetyldodecanol, 2-butyloctanol, 2-hexyldecanol, 2-undecylpentadecanol or oleyl alcohol; fluoro oils, optionally partially hydrocarbon-based and/or silicone-based; silicone oils, for instance volatile or non-volatile, linear or cyclic polydimethylsiloxanes (PDMSs); polydimethylsiloxanes comprising alkyl, alkoxy or phenyl groups, which may be pendent or at the end of a silicone chain, these groups comprising from 2 to 24 carbon atoms; phenyl silicones, for instance phenyl trimethicones, phenyl dimethicones, phenyl trimethylsiloxy diphenyl siloxanes. diphenyl dimethicones, diphenyl methyldiphenyl trisiloxanes and 2-phenyl ethyl trimethyl siloxysilicates; and mixtures thereof.

In general, any natural or synthetic oil for cosmetic use is suitable for the composition of present invention. Non-limiting examples of such as esters (e.g., alkyl benzoates having between12 to 15 carbons), triglycerides (e.g., Caprylic/Caprytate triglyceride), hydrocarbons (e,g., mineral oil, Petroleum Jelly), natural oils (e,g., Jojoba Oil, Safflower Oil, Olive Oil, Sweet Almond Oil, Mango Butter, Sunflower Oil, Aloe Butter, Cocoa Butter, Shea Butter, Palm Kernel Flakes, Beeswax, Lanolin Butter. Sweet Orange Oil, Lemon Oil, Avocado oil, Coconut Oil, Palm Oil. Sesame Oil, Peanut Oil, Peach Kernel Oil, Wheat Germ Oil, Macadamia Nut Oil, Night Primrose oil, Soya Oil and the derivatives thereof.), and castor oil, among others. Suitable oils are also disclosed, for example, at column 3, line 37, to column 4, line 4, of U.S. Patent No. 5.738.125: hereby incorporated by reference. Silicone oils may also be used as cosmetic oils.
Suitable synthetic oils are at least one member selected from the group consisting of fatty alcohols; fatty acid esters such as isopropyl myristate, palmitate, stearate and isostearate; oleyl oleate; isocetyl stearate; hexyl laurate; dibutyl adipate; dioctyl adiphate; myristyl myristate; oleyl erucate; polyethylene glycol and it derivatives; polyglyceryl fatty acid esters; and cetyl palmitate, by way of example only.
Silicone oils are also suitable. Useful silicone oils are non-volatile silicone oils known by INCl names that include dimethicone or dimethiconol, Volatile silicone oils such as cyclomethicones may also be used.

Typical amounts of the oil/emoillent are about 1 to about 50 wt%, in some cases about 5 to about 40wt.% and in some cases about 15 to about 30wt.%, depending on the amount of active ingredient that is encapsulated Solvents

In another aspect of the invention, the composition comprises at least one of water and water soluble solvents. Embodiments according to the invention include water and solvents from, but not limited to, the group of naturally-derived or synthetic alcohols, naturally-derived or synthetic glycols and naturally-derived or synthetic polyols, including triols, wherein alcohols comprise primary, secondary and tertiary alcohols that are commonly used and accepted in cosmetic formulations and compositions. Alcohols and glycols accepted in cosmetic products include, but are not limited to, ethanol, 1,2-propanediol, 1,3-propanediol, 1,3-butylene glycol, and polyols accepted in cosmetic products include, but are not limited to, glycerol. In one aspect of the invention, the solvent comprises lower monoalcohols comprising from 1 to carbon atoms, such as ethanol or isopropanol, glycols comprising from 2 to 8 carbon atoms, such as propylene glycol, ethylene glycol, 1,3-butyleneglycol or Dipropylene glycol, C∼-C4ketones and C∼-C4 aldehydes.

According to further embodiments according to the invention the solvents comprising naturally-derived or synthetic alcohols may be combined with naturally-derived or synthetic glycols and/or naturally-derived or synthetic polyols in weight ratios (alcohol: glycol and/or polyol) from about 1 : 0.1 to about 1 : 10, in some cases from about 1 : 0.4 to about 1 : 6, and other cases from about 1 : 0.6 to about 1 : 4, and in yet other cases from about 1 : 1 to about 1 : 2.

Typical amounts are about 1 to about 30 wt%, in some cases about 1 to about 20wt.% and in some cases about 1 to about 15wt.% of the composition.

### Active ingredients

The encapsulated lipophilic or oil soluble active ingredient or oil-miscible or oil-dispersible active ingredient can be chosen from cosmetically suitable antioxidants, vitamins, plant extracts. synthetic materials with e.g. anti aging, skin soothing, skin tone, anti cellulite, anti inflammatory, whitening, improving overall skin condition properties. The lipophilic active ingredient comprises at least retinol.

Further optional lipophilic or oil soluble or oil miscible or oil dispersible active ingredients and lipophilic or oil soluble or oil miscible or oil dispersible extracts might be selected from: Acacia famesiana extract, Acacia senegal, Açai, Acerola fruit extract, Acetyl carnitine HCL, Acetyl glucosamine, Acetyl hexapeptide-8, Acetyl octapeptide-3, Achillea millefolium, Acne soap, Actaea racemosa, Adenine, Adenosine, Adenosine triphosphate, Aerocarpus santalinus, Aesculus hippocastanum, Agaricus bisporus extract, Agrimonia eupatoria leaf extract, Ahnfeltia concinna extract, Ahnfeltia extract, Ajuga turkestanica extract, Alanine, Alaria esculenta, Alchomilla vulgaris, Aleurites moluccana seed oil, Alfalfa extract, Algae extract, Algin, Allanotin Panthenol, Allantoin, Allantoin Ascorbate, Allantoin Biotin, Allantoin Galacturonic Acid, Allantoin Glycyrrhetinic Acid, Allantoin Polygalacturonic Acid, All-trans retinoic acid, Aloe barbadensis, Aloe barbadensis leaf juice extract, Aloe barbadensis leaf juice powder, Aloe extract, Aloe vera, Alpha bisabolol, Alpha lipoic acid, Alpha-, beta-, gamma-, and delta-Tocopherol, Alteromonas ferment extract, Althaea rosea, Althea officinalis, Amino acids, Aminobutyric acid, Amygdalic acid, Amyris oil, Anacyclus pyrethrum, Anacystis nidulans extract, Ananas sativus fruit extract, Andiroba oil. Anethum graveolens, Angelica archangelica root oil, Angelica polymorpha sinensis root extract, Anise, Annato extract, Anthemis nobis flower extract, Apple fruit extract, Apricot kernel oil, Aquaporins, Arachis hypogaea extract, Arbutin, Arctium lappa, Arctostaphylos uva ursi leaf, Argan oil, Argania spinosa, Arginine, Argireline, Arnica extract, Artemia extract, Artemisia absinthium extract, Artemisia vulgaris, Ascophyilum nodosum, Ascorbic acid, Ascorbyl Dipalmitate, Ascorbyl glucosamine, Ascorbyl glucoside, Ascorbyl methylsilanol pectinate, Ascorbyl Palmitate, Ascorbyl palmitate, Asparagine, Asparagopsis armata extract, Aspartic acid, Astragalus membranaceus, Astragalus sinicus, Astrocaryum murumuru seed butter, Atractyloydes lancea root extract, Avena sativa, Average, Avocado oil (persea gratissima). Awapuhi, Azadirachta indica, Babassu oil, Bakuchiol, Balm mint extract, Balm oil, Balsam peru, Banana extract, Barberry, Barley extract, Bay leaf oil. Beeswax / Cera Alba, Beet root extract, Behenic acid, Bellis perennis, Berberis aristata, Bergamot oil, Bertholletia excelsa extract, Beta and alpha Bisabolol, Beta hydroxy acid, Beta vulgaris root extract, Seta-carotene (provitamin A), Beta-sitosterol, Betula alba. BHA (beta hydroxy acid), Bilberry extract, Bioflavonoid, Biotin, Birch bark extract, Birch leaf extract, Bisabolol, Bitter orange flower, Black cohosh, Black currant oil. Black elderberry, Black locust extract, Black mulberry, Black pepper extract and oil, Black raspberry, Black tea, Blackberry, Bladderwrack extract, Bloodwort, Boerhavia diffuse root extract, Bois de rose oil, Bois oil, Borage seed extract, Borage seed oil, Borago officinalis extract, Borago officinalis seed oil, Boswellia carterii, Boswellia serrata extract, Boysenberry, Brassica campestris, Brazil nut extract, Bromelain, Brown algae, Buddleja davidii extract, Bupleurum falcatum extract, Burdock root, Butcher's broom extract, Butyrospermum Parkii, Buxus chinensis, Caffeine, Cajeputi oil. Calcium ascorbate, Calcium gluconate, Calcium pantothenate, Calendula extract, Calendula officinalis flower extract, Calluna vulgaris flower extract, Calophyllum inophyllum seed oil, Camellia japonica, Camellia oleifera, Camellia sinensis, Cananga extract, Cananga odorata, Candelilla wax, Cannabis sativa L. Oil, Canola oil, Capsaicin, Capsicum, Capsicum oleoresin, Caraway oil, Carmine, Carnitine, Carrot oil. Carthamus tinctorius oil, Carya illinoensis oil, Castanea sativa seed extract, Castor oil, Catalase, Caulerpa taxifolia extract, Coderwood, Celandine, Centaurea cyanus, Centalla asiatica. Ceresine Chamomile, Chamomile oil, Chamomilla recutita flower extract, Chaparral extract, Chaulmoogra oil, Cherry extract, Chitosan, Chlorella, Cholecalciferol, Cholesterol, Choline, Chondroitin sulfate, Chondrus crispus, Chrysanthemum extract, Chrysanthemum parthenium extract, Cichorium intybus, Cimicifuga racemosa root extract, Cinnamomum, Cinnamomum camphora, Cinnamon, Citrullus colocynthis, Citrus amara, Citrus aurantifolia, Citrus aurantium, Citrus aurantium extract, Citrus medica limonium, Citrus unshiu, Citrus unshlu peel extract, Clary oil, Clove leaf, Clove oil, Clover blossom, Clover leaf oil, Cocoa butter, Cocoa extract, Coconut, Coconut oil, Coconut oil-derived fatty acids, Cocus nucifera, Codium tomentosum extract, Coenzyme Q10 (ubichinone), Coffea arabica (coffee) seed oil, Coffea arabica extract, Cola acuminata seed extract, Coleus barbatus, Collagen amino acid, Coltsfoot, Comfrey extract, Commiphora myrrha extract, Coneflower, Copaifera officinalis. Copper gluconate. Copper peptides, Corallina officinalis extract, Com Acid, Corn Glycerides, Corn oil, Commin, Comus extract, Corylus americana, Corylus avellana, Coumarin, Cranberry seed extract, Cranberry seed oil, Crataegus monogina extract, Creatine, Creatinine, Cucumber extract, Cucumis melo (melon) fruit extract, Cucumis sativus extract, Cucurbita pepo seed extract, Cucurbitea peponis, Curcuma longa root, Curcumin, Cyamopsis tatraganoloba, Cyanocobalamin, Cymbopogon citrates, Cymbopogon martini, Cysteine, Cystine, Daisy flower extract, Dandelion extract, Daucus carota, Decarboxy camosine HCL,Diethylhexyl syringylidenemalonate, Dill extract,Dioleyl Tocopheryl Methylsilanol, Dioscorea villosa extract. Dipotassium glycyrrhizate, Dipotassium glycyrrhizinate, Dog rose, Dogwood, Dong qual, D-panthenol, Dulse, Durien, Durvillaea antarctica extract, Eicosapentaenoic acid, Elaeis guineensis, Elastin, Elderberry, Elecampane, Ellagic acid, Emp seed oil, English ivy extract, Enteromorpha compressa extract, Enzymes, Epidermal growth factor (EGF), Epigallocatechin gallate, Epilobium angustifolium extract, Equisetum arvense, Ergocalciferol, Ergothioneine, Eriobotrya japonica, Escin, Ester-C, Ethyl macadamiate, Ethyl vanillin, Ethylhexyl Myristate, Eucalyptus oil, Eugenia aromatica, Eugenia caryophyllus, Euphorbia Cerifera (Candelilla) Wax, Euphorbia cerifera wax, Euphrasia officinalis, Euterpe oleraca, Evening primrose oil, Evodia rutaecarpa extract, Fagus sylvatica extract, Farnesol, Farnesyl acetate, Fennel oil, Fennel seed extract, Ferula foetida, Ferula galbaniflua, Ferulic acid, Feverfew extract, Fibronectin. Ficus carica fruit extract. Fig, Filipendula glaberrima, Filipendula rubra, Fir needle oil, Fireweed extract, Flavonoid, Flax, Flaxseed oil, Foeniculum vulgare extract, Folic acid, Fu ling, Focus vesiculosus extract, Galactoarabinan, Gamma linolenic acid (GLA). Ganoderma lucidum extract, Gardenia florida extract, Gellidiela acerosa extract, Genistein, Gentiana lutea (Gentian) root extract, Geranium extract, Geranium oil, Ginger extract, Ginger oil. Ginkgo biloba leaf extract, Ginseng, GLA, Glabridin, Glutamine, Glutathione, Glycine soja oil, Glycine soja seed extract, Glycine soja sterols, Glycogen, Glycol Ricinoleate, Glycolipid, Glycoproteins, Glycosphingolipid, Glycyrrhetic acid, Glycyrrhiza glabra, Glycyrrhiza uratensis root extract, Goldenseal, Gossypium herbacuem seed oil, Gotu kola, Grape seed extract, Grape seed oil, Grapefruit oil, Grapefruit peel extract, Green tea, Gromwell, Guaiac wood, Guaiacum officinale, Guarana, Haematococcus pluvialis extract, Hamamelis virginiana, Hamamelitannin, Haslea ostrearia extract, Hawthorn extract, Hayflower extract, Hedera helix, Helianthus annuus seed oil, Helianthus oil, Hepatocyte growth factor (HGF), Hesperidin, Hesperidin methyl chalcone, Hexylresorcinol, Hibiscus. Himanthalia elongate extract, Hippophae rhamnoides, Histidine, Hops, Hordeum vulgare extract. Horse chestnut extract, Horse elder, Horseradish, Horsetail extract, Hortonia floribunda leaf extract, Huang qi, Humulus lupulus extract, Hyaluronic acid, Hydnocarpus anthelmintica, Hydrastis canadenis, Hydrogenated olive oil, Hydroxyproline, Hypericum extract, Hyssop, idebenone, Ilex paraguariensis, Illicium vemum, Inositol, Insulinlike growth factor (IGF), Inula helenium, loflavones, Iris florentina extract, Irish moss extract, Isoflavone, Isoleucine, Isotretinoin, Isotridecyl Myristate, Ivy extract, Jasmine oil, Jasminium grandiflorum, Jewelweed, Jojoba butter, Jojoba esters, Jojoba oil, Jojoba wax, Jonquil extract, Ju hua, Jujube fruit extract, Juniper berry, Juniperus communis, Kava-kava extract, Kawa extract, Kelp extract, Kigelia africana extract, Kinetin, Kiwi fruit extract, Kola nut, Kudzu root, Kukui nut oil, Lactobionate, Lactoperoxidase, Lady's mantle extract, Lady's thistle (milk thistle) extract, Lagerstroemia indica extract, Laminaria digitata, laminaria longicruris, Laminaria ochroleuca extract, Laminaria saccharine, Lamium album flower extract, Lappa extract, Larrea divaricata extract, Larrea tridentata, L-ascorbic acid, Lauric acid, Laurus nobilis, Lavandin oil, Lavandula angustifolia, Lavandula officinalis, Lavender extract and oil, L-carnitine, L-cysteine Lemon, Lemon oil, Lemongrass extract, Lemongrass oil, Lentil fruit extract, Lentinus edodes extract, Leontopodium alpinum extract, Leptospermum scoparium oil, Leucine, Levisticum officinale root extract, Licorice extract, Licorice root, Lilium candidum bulb extract, Lime oil and extract, Limnanthes alba, Limnanthes alba seed oil, Linden flower extract, Linoleic acid, Linolenic acid, Linseed oil, Linum usitatissimum extract, Lithospermum erythrorhizon, Litsea cubeba, Locust bean, Lonicera caprifolium flower extract, Lonicera japonica, Lonicera japonica flower extract, Loquat extract, Lotus seed extract, Lovage root extract, Lutta cylindrica seed oil or extract, Lupine, Lupine oil, Lupinus albus extract, Lutein, Lycium barbarum fruit extract, Lycopene, Lycosaminoglycans, Lysine, Macadamia nut oil, Macadamia nut oil (macadamia temifolia), Madecassoside, Magnesium ascorbyl palmitate, Magnesium ascorbyl phosphate, Magnesium gluconate. Malva sylvestris extract, Malvaceae extract, Mandarin orange oil or extract, Manganese gluconate, Mangifera indica (mango) seed butter, Mangifera indica root, Manuka oil, Mangold, Marionberry, Marjoram, Marshmallow, Mastocarpus stellatus, Mate extract, Matricaria flower extract, Matricaria oil, Meadowfoam seed oil, Meadowsweet extract, Medicago sativa, Melaleuca afternifolla, Melaleuca cajeputi oil, Melia azadirachta, Melibiose, Melissa officinalis, Mentha arvensis, Mentha piperita, Mentha spicata, Mentha viridis, Menthol, Methylsufonylsulfate, Microcystis aeruginosa, Milk protein, Milk vetch root, Millet seed extract, Mimosa oil or extract, Mint, Mitracarpe scaber extract, Mixed fruit extracts, Morinda citrifolia, Morus bombycis root extract, Morus nigra root extract, Mugwort extract, Mulberry extract, Murumuru seed butter, Myrtle extract, Myrtus communis extract, N6-furfuryladenine, N-acetyl-L tyrosine, Narcissus poeticus wax, Nardostachys jatamansi, Nasturtium officinale extract, Natural moisturizing factor (NMF), Neem extract, or oil, Neroli. Neroli oil, Nettle extract, Nordihydroguaiaretic acid, Nymphaea tetragona, Oak root extract, Oat bran extract, Oat kernel extract, Oatmeal, Oenothera biennis oil, Olea europaea fruit oil, Olea europaea oil unsaponifiables, Olive oil/olive fruit oil, Olive oil-derived fatty acids, Orange blossom, Orbignya martiana, Orbignya oleifera, Orchid, Origanum majorana, Origanum vulgare flower extract, Orris root, Oryza Sativa (Rice) Bran Oil, Oryza Sativa (Rice) Extract, Oryza Sativa (Rice) Germ Oil, Oryza sativa oil, Oryzanol, Oxidoreductase, Ozokerite, P. Elisabethae, Padina pavonica extract Paeonia albiflora extract, Paeonia suffruticosa extract, Palmaria palmata extract, Palmarosa oil, Palmitoyl hexapeptide-12, Palmitoyl oligopeptide, Palmitoyl pentapeptide-3, Palmitoyl tetrapeptide-7, Palmitoyl tripeptide-1, Panax ginseng root extract, Panicum miliaceum, Pansy extract, Pantethine, Panthenol, Pantothenic acid, Papain, Papaver somniferum seed, Papaya extract, Passiflora edulis extract, Passiflora edulis seed oil. Passion fruit extract, Patchouli, Paullinia cupana seed extract, Pawpaw extract, Peanut oil, Pecan oil, Pectin, Pelargonium graveolens oil, Pellitory, Peony flower, Peony root extract, Peppermint, Perilla ocymoides, Persea gratissima oil, Persicaria hydropiper, Petitgrain mandarin, Phenylalanine, Phenylethyl resorcinol, Phloretin, Phyllanthus emblica fruit extract, Phytic acid, Phytoestrogen, Phytonadione, Phytosphingosine, Phytosterol, Picea excelsa extract, Pine oil, Pineapple extract, Pinecone extract, Pinus lambertiana wood extract, Pinus sylvestris extract, Piper nigrum. Pistachio seed oil, Pistacia vera seed oil, Pisum sativum, Plankton extract, Plectranthus barbatus extract, Plum extract, Plum oil, Plum seed oil, Pogostemon cablin, Polygonum cuspidatum root extract. Pomegranate extract. Poria cocos extract, Porphyridium cruentum extract, Portulaca oleracea extract, Potassium Ascorbyl Tocopheryl Phosphate, Potassium Cornate, Proline, Propolis, Prunella vulgaris, Prunus americana, Prunus amygdalus dulcis, Prunus armeniaca, Prunus domestica seed extract, Prunus domestica seed oil, Pseudopterogorgia efisabethae, Pueraria lobata, Pullulan, Pumpkin, Pumpkin seed extract, Punica granatum extract, Pyonogenol, Pyridoxine hydrochloride (HCL), Pyrus cydonia, Pyrus malus, Quercetin, Quercus, Quercus infectoria extract, Quillaja extract, Quince seed, Quinoa oil, Ranunculus ficaria extract, Rapeseed oil, Raspberry seed extract, Raspberry seed oil, Red algae, Red clover, Red raspberry extract, Red sandalwood, Renova, Resorcinol, Resvanatrol, Ratin-A, Retinoids, Retinol palmitate, Retinyl ascorbate, Retinyl palmitate. Retinyl retinoate, Rhus Succedanea Fruit Wax, Riboflavin, Rice bran oil, Rice oil, Ricinoleic. Acid, Ricinus communis, Ricinus Communis (Castor) Seed Oil, Robinia pseudacacia extract, Rosa canina, Rosa centifolia, Rosa centifolia flower, Rosa damascena oil, Rosa eglanteria, Rosa gallica flower extract, Rosa mosqueta, Rosa roxburghii extract, Rosa rubiginosa, Rose flower, Rose flower oil, Rose hip, Rose hip oil, Rose oil, Rosemary extract, Rosemary oil, Rosemary oil, Rosewood oil, Rosmarinus officinalis extract, Rubus idaeus, Rubus occidentalis, Rubus ursinus, Rubus villoscus, Ruscogenine, Ruscus aculeatus, Rutin, Sage extract, Salix alba extract, Salix nigra (willow) bark extract, Salvia officinalis, Sambucus canadensis, Sambucus cerulea, Sambucus nigra, Sandalwood oil, Santalum album seed extract, Sapindus mukurossi peel extract, Saponaria officinalis extract, Saponin, Sargassum filipendula extract, Sausurrea oil, Saw palmetto extract, Saxifrage sarmentosa extract, Sclareolide, Scutellaria balcalensis extract, Sea buckthorn, Sea whip extract, Seaweed, Serenoa serrulata extract, Sericin, Serine, Sesame oil, Sesamum indicum, Shao-yao, Shea butter, Siegesbeckia orientalis, Silk protein, Silver tip white tea leaf extract Silybum marianum extract, Simmondsia chinensis, Skullcap extract, Slippery elm bark, Soapberry extract, Soapwort, Sodium ascorbate, Sodium ascorbyl phosphate, Sodium chondroitin sulfate, Sodium dilauramidogfutamide lysine, Sodium hyaluronate, Sodium PCA, Sodium Potassium Sodium Ricinoleate, Solanum lycopersicum extract, Sonojell, Soy extract, Soy isoflavones, Soy oil, Soy protein, Spearmint oil, Spikenard, Spiraea ulmaria, Spirulina, Squalane, Squalane, St. John's wort, Star anise, Stearyl glycyrrhetinate, Stem cells, Strawberry begonia, Sucrose, Sugarcane extract, Sunflower seed oil, Superoxide dismutase. Sweet almond, Symphytum officinale extract, Tamanu oil, Tamarindus indica seed extract, Tanacetum parthenium, Tangerine oil, Taraktogenos kurzii, Taraxacum officinale, Tazorac, Tea tree oil, Tea tree oil, Terminalia catappa, Terminalia sericea, Terminalia sericea extract, Theobroma cacao seed butter, Thiamine HCL, Thiotaurine, Threonine, Thyme extract, Thyme oil, Thymus serpillum extract, Thymus vulgaris, Thymus vulgaris oil, Tilia cordata, Tocopherol, Tocopherol acetate, Tocopheryl Acetate, Tocopheryl lineolate, Tocopheryl Linoleate, Tocopheryl Linoleate/Oleate, Tocopheryl Nicotinate, Tocopheryl Succinate, Tocotrienols, Tomato extract, Tormentil extract, Tridecyl Myristate, Trifolium pratense, Trigonella foenum-graecum seed extract, Trihydroxystearin, Trilinolein, Tripalmitin. Triticum vulgare (wheat) germ extract, Triticum vulgare oil, Troxerutin, Tryptophan, Tussilago farfara, Ubiquinone, Ulmus fulva bark extract, Ulva lactuca extract, Uncaria tomentosa extract, Undaria pinnatifida, Urea, Urtica dioica, Uva ursi extract, Vaccinium macrocarpon fruit extract, Vaccinium myrtillus, Valine, Vanilla planifolia fruit extract, Verbena extract, Vetiver oil or extract, Viola tricolor extract, Visnaga vera extract, Vitamin A, Vitamin D, Vitamin E, Vitamin F, Vitis vinifera, Vitreoscilla ferment, Walnut extract, Walnut oil, Watercress extract, Watermelon fruit extract, Wheat germ oil, Wheat germ oil (triticum vulgare), Wheat protein, White nettle, White oak bark extract, White tea leaf extract. White willow, Wild ginger, Wild yam extract, Willow bark, Willow herb, Wintergreen oil, Witch hazel, Xanthophyll, Xi xin, Ximenia americans oil, Yarrow extract, Yeast, Yeast extract, Verba mate extract, Ylang ylang, Yucca extract, Zanthoxylum piperitum, Zea Mays (Corn) Silk Extract, Zinc Myristate, Zingiber officinale roscoe, Zingiber zerumbet, Zingiberaceae, Zizyphus jujuba fruit extract, C 2 -C 18 esters of RETINOL, vitamin E, esters of vitamin E, 3,4-didehydro-RETINOL, plant extracts/essential oils, ursolic acid, oleanolic acid, oil-soluble licorice, and lipoic acid.

Typical amounts are about 1 to about 50 wt%, in some cases about 1 to about 25wt.% and in some cases about 1 to about 10wt.%, depending on physical stability results.

### Auxilliary Materials

The Auxilliary Materials are typically water soluble and present in the water phase surrounding the encapsulated lipophilic core.

Chelating Agents: Optionally in certain embodiments of the invention the composition according to the invention comprises at least one chelating agent. The composition may contain less than about 5%, or from about 0.01 to about 3% of a chelant such as citrates, nitrogen-containing, P-free aminocarboxylates such as EDDS, ethylenediamintetraacetic acid (EDTA) and its salts e.g. disodium EDTA; DTPA; aminophosphonates such as diethylenetriamine pentamethylenephosphonic acid and ethylenediamine tetramethylenephosphoric acid; nitrogen free phosphonates e.g., HEDP; nitrilotriacelic acid (NTA) and its salts; salts and esters of oxalic acid, tartaric acid, phytic acid, glutamic acid and its salts and preferably N,N-bis(carboxymethyl)glutamic acid and its salts and their blends; and nitrogen or oxygen containing, P-free carboxylate-free chelants such as compounds of the general class of certain macrocylic N-ligands.

Typical amounts are about 0,01 to about 2 wt%, and preferably about 0,01 to about 0.5 wt% depending on physical stability results.

Preservatives: Optionally in certain embodiments of the invention the composition according to the invention comprises at least one preservative. According to further embodiments of the foregoing, the at least one preservative is selected from but not limited to the group consisting of ethylparaben; imidazolidinyl urea; methylpareben, sorbic acid and its salts, O- phenylphenol; propylparaben: quatemium-14; quatemium- 5: sodium dehydroacetate; phenoxyethanol, phenoxyisopropanol, benzyl alcohol, polyaminopropyl biguanide, triethylene glycol, piroctone olamine, benzoic acid and its salts, dehydroacetic acid and its salts, diazolidinyl urea, iodopropynyl butylcarbamate, methylisothiazolinone, glyceryl caprylate, phenylethylalcohol, caprylyl glycol, capryloyl glycine, phenylpropanol, ethylhexylglycerin, ethylpropanediol, and their salts and their blends,

In general, the preservatives may be used in any amount which is effective to prevent or retard microbial growth and typically about 0.01 wt% to about 5 wt%, and in some cases from about 0.05 wt% to about 3 wt%, based on the total weight of the composition.

Gelling and Stabilizing Agents: The gelling agents which can be used in the composition according to the invention can be polymeric gelling agents or organic lipophilic gelling agents. The polymeric or organic lipophilic gelling agents are partially or completely crosslinked or non crosslinked but able to create a three dimension structure such as Xanthan Gum, polyamide, silicone polyamides, silicone crosslinked polymers and acrylates including poly C10-30 alkyl acrylates and side-chain crystalline polymers (e.g., Example 23). Gelling agents can be present in the composition according to the invention in a content ranging from 0.1 to 10% by weight, preferably from 0.5% to 7% and most preferably 0.01 to 1%.

The inventive composition can be used in a wide range of Personal Care products. The inventive composition typically comprises about 0,5 % to about 50 % and preferably about 2 to about 10 % of the Personal Care products. The inventive composition can be combined with other ingredients of a Personal Care product by using conventional equipment and methods.

Certain aspects of the instant invention are illustrated by the following Examples.

### Example Compositions

### Example 1:

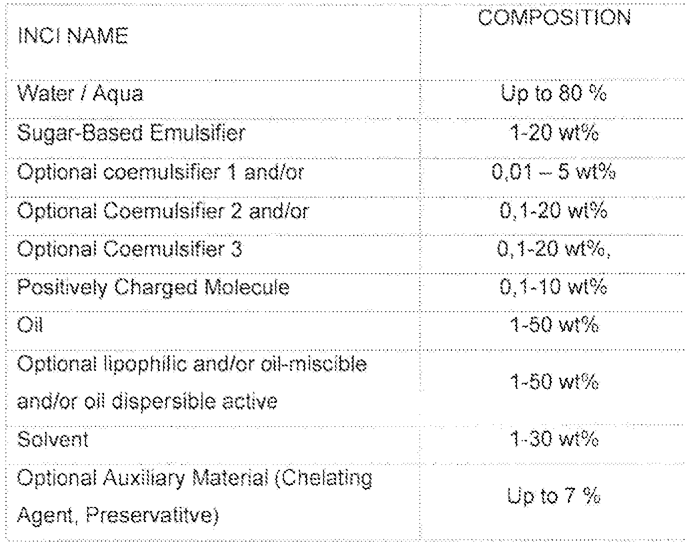

### Manufacturing

Water Phase consisting of water, sugar based emulsifier, at least one coemulsifier, positively charged molecule and optional auxiliary material are premixed in a stainless steel vessel at a temperature depending on the instructions given in the technical information of the chosen ingredients. This can vary from RT to 90 °C. The ingredients of the oil phase constising of optional lipophilic active and/or optional oil and or optional coemulsfier are mixed at a temperature from RT to 90°C in a stainless steal vessel and added to the water phase while stirring followed by homogenization prozess with commercially available equipment (homogenization at high shear rates and/or high pressure homogenization from 100-1000 bar and typically about 600 bar and at a temperature ranging from about room temperature to less than 100°C; depending on the composition ingredients).

### Example 2:

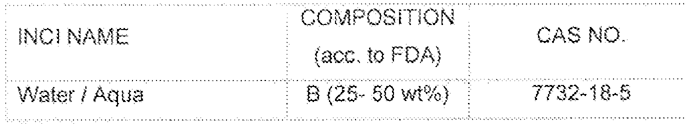

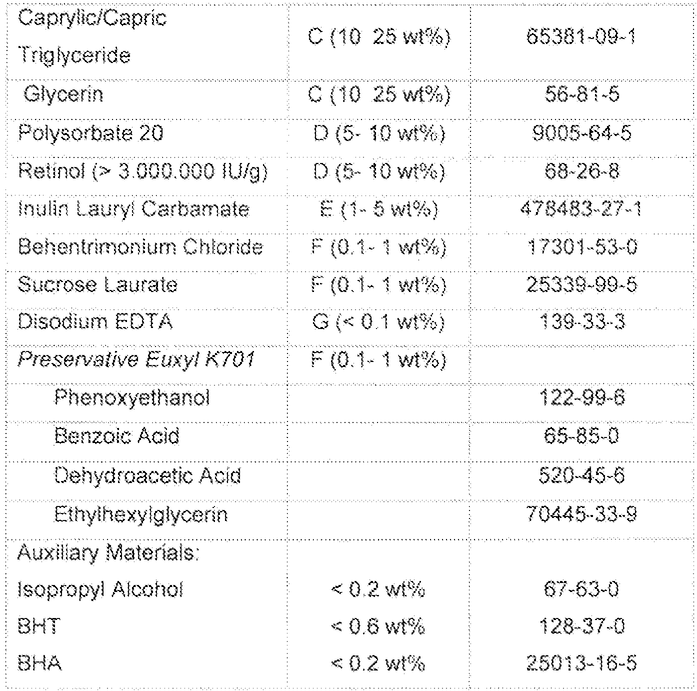

### Manufacturing

Water Phase consisting of water, sugar based emulsifier, coemulsifiers, positively charged molecule and auxiliary materials are premixed in a stainless steel vessel at a temperature of 60°C, after that cooled to RT. The ingredients of the oil phase constising of lipophilic active and oil are mixed at RT in a stainless steel vessel and added to the water phase while stirring followed by homogenization prozess with commercially available equipment (homogenization at high pressure homogenization from 100-1000 bar and typically about 600 bar at RT.

### Example 2a:

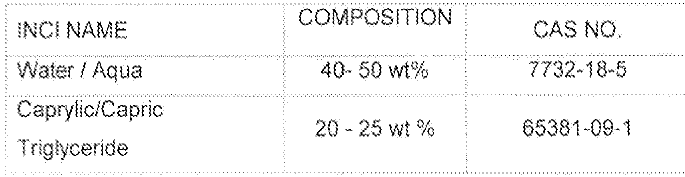

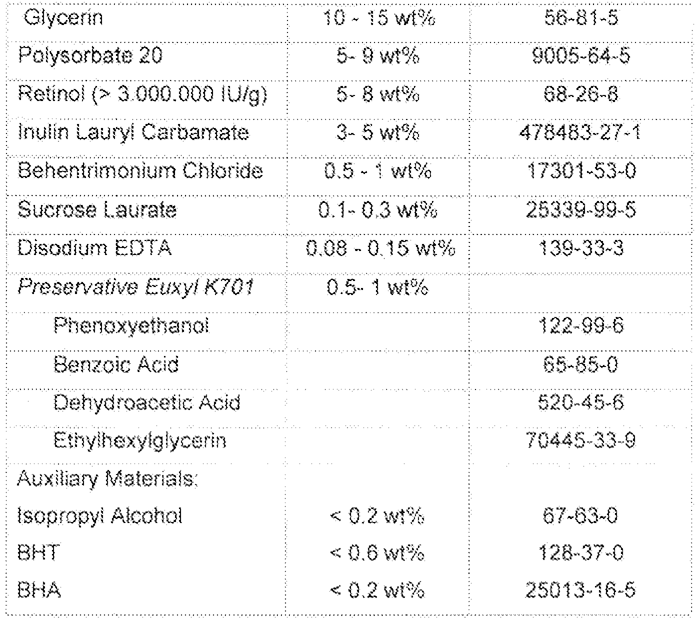

### Manufacturing

Water Phase consisting of water, sugar based emulsifier, coemulsifier 1, positively charged molecule and auxiliary materials are premixed in a stainless steel vessel at a temperature of 60°C, after that cooled to RT. The ingredients of the oil phase constising of lipophilic active, coemulsifier 2 and oil are mixed at RT in a stainless steel vessel and added to the water phase while stirring followed by homogenization prozess with commercially available equipment (homogenization at high pressure homogenization from 100-1000 bar and typically about 600 bar at RT.

### Example 2b:

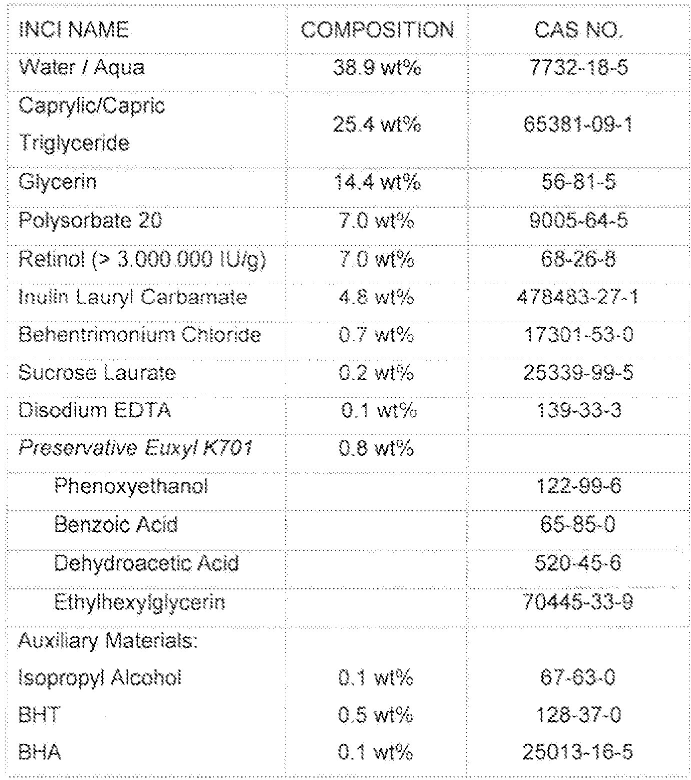

### Manufacturing

Water Phase consisting of water, inulin lauryl carbamate, sucrose laurate, behentrimonium chloride, isopropyl alcohol, glycerin were premixed in a stainless steel vessel at a temperature of 60°C, after that cooled to RT. The ingredients of the oil phase constising of retinol, caprylic/capric triglyceride, BHA, BHT and polysorbate 20 were mixed at RT in a stainless steel vessel and added to the water phase while stirring followed by homogenization prozess with commercially available equipment (homogenization at high pressure homogenization of about 600 bar at RT. The particle size of the physically stable example was measured to be 64.6 nm and the zeta potential was +40-50 mV.

### Example 2c:

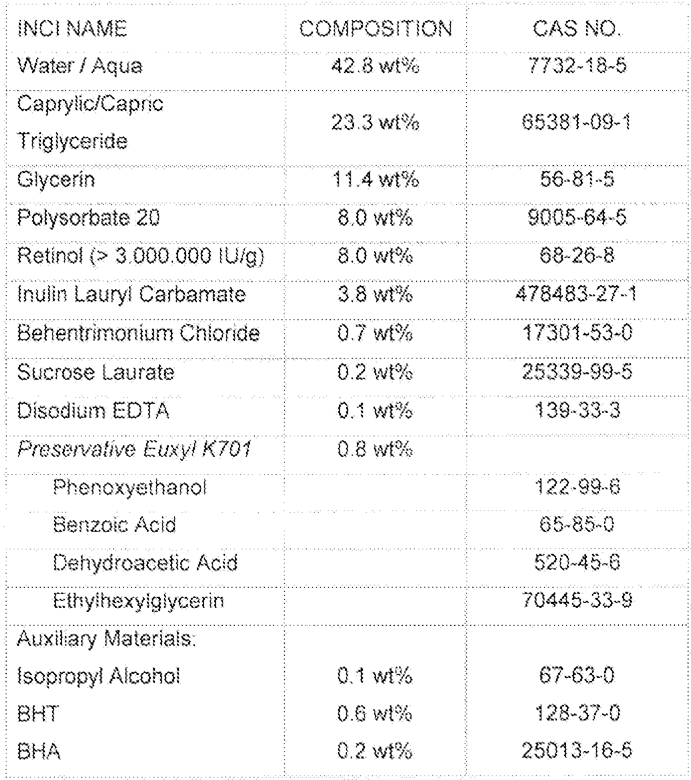

Water Phase consisting of water, inulin lauryl carbamate, sucrose laurate, behentrimonium chloride, isopropyl alcohol, glycerin were premixed in a stainless steel vessel at a temperature of 60°C, after that cooled to RT. The ingredients of the oil phase cohstising of retinol, caprylic/capric triglyceride, BHA, BHT and polysorbate 20 were mixed at RT in a stainless steel vessel and added to the water phase while stirring followed by homogenization prozess with commercially available equipment (homogenization at high pressure homogenization of about 600 bar at RT. The particle size of the physically stable example was measured to be 78.5 nm and the zeta potential was approximately +43 mV (alternatively: +40-50mV as given in previous samples).

### Example 3 (not part of the invention as claimed)

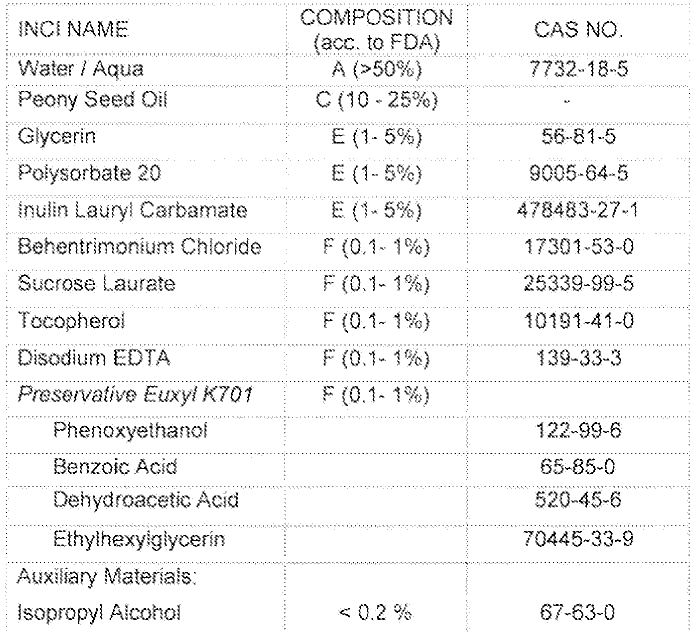

### Manufacturing

Water Phase consisting of water, sugar based emulsifier. coemulsifiers, optional positively charged molecule and auxiliary materials are premixed in a stainless steal vessel at a temperature of 6.0°C, after that cooled to RT. The ingredients of the oil phase constising of lipophilic active and oil are mixed at RT in a stainless steal vessel and added to the water phase while stirring followed by homogenization prozess with commercially available equipment (homogenization at high pressure homogenization from 100-1000 bar and typically about 600 bar at RT.

### Example 4:

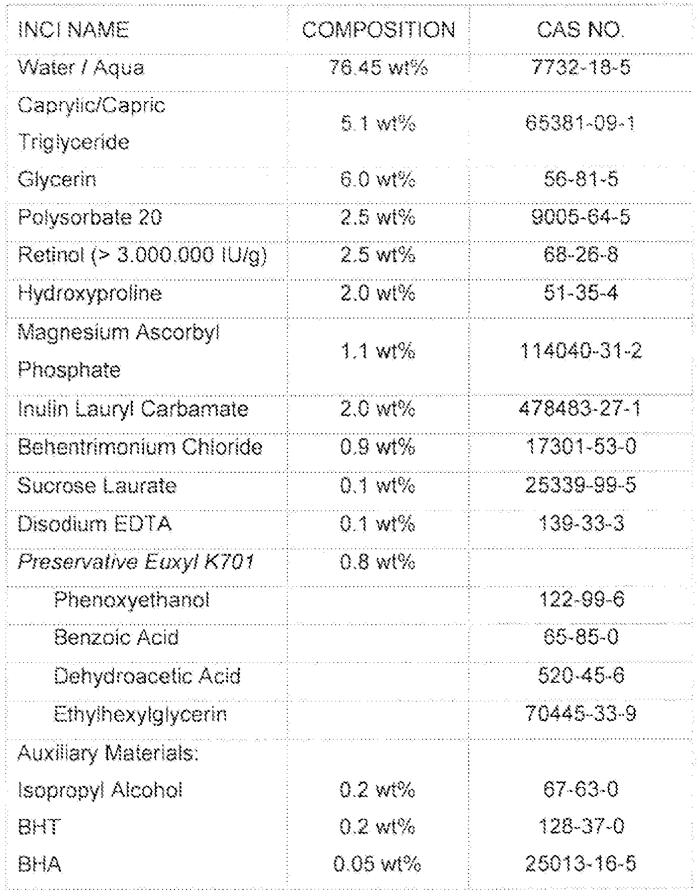

### Manufacturing

Water Phase consisting of water, inulin lauryl carbamate, sucrose laurate, behentrimonium chloride, isopropyl alcohol, glycerin, hydroxyproline and magnesium ascorbyl phosphate were premixed in a stainless steel vessel at a temperature of 60°C, after that cooled to RT. The ingredients of the oil phase constising of retinol, caprylic/capric triglyceride, BHA, BHT and polysorbate 20 were mixed at RT in a stainless steel vessel and added to the water phase while stirring followed by homogenization prozess with commercially available equipment (homogenization at high pressure homogenization of about 600 bar at RT. The particle size of the physically stable example was measured to be 113.3 nm and the zeta potential was +20-30mV.

### Example 5:

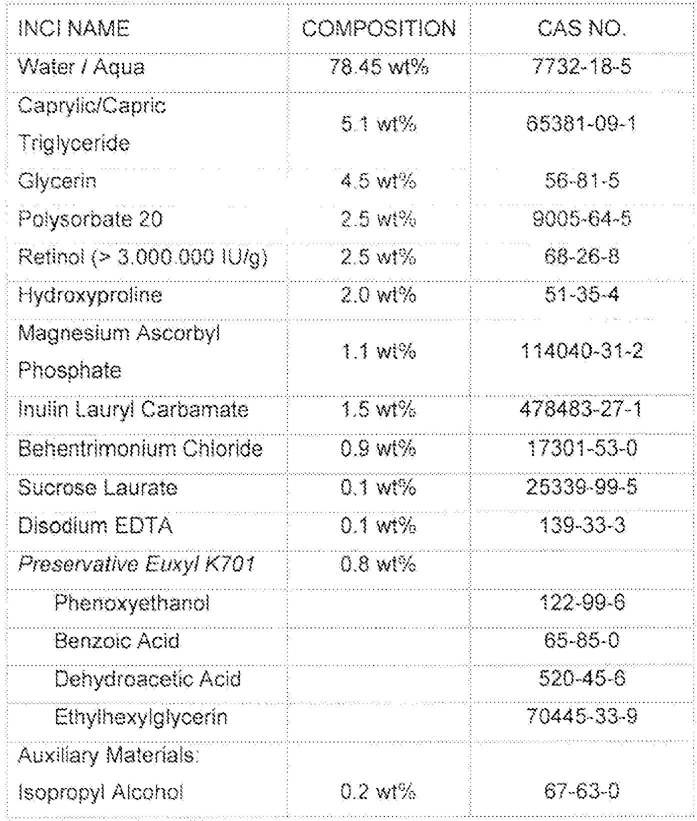

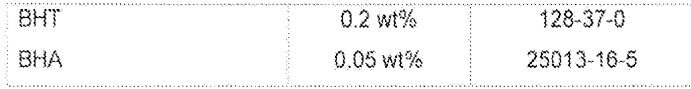

### Manufacturing

Water Phase consisting of water, inulin lauryl carbamate, sucrose laurate, behentrimonium chloride, isopropyl alcohol, glycerin, hydroxyproline and magnesium ascorbyl phosphate were premixed in a stainless steel vessel at a temperature of 60°C, after that cooled to RT. The ingredients of the oil phase constising of retinol, caprylic/capric triglyceride, BHA, BHT and polysorbate 20 were mixed at RT in a stainless steel vessel and added to the water phase while stirring followed by homogenization prozess with commercially available equipment (homogenization at high pressure homogenization of about 600 bar at RT. The particle size of the physically stable example was measured to be 94.1 nm and the zeta potential was +23.2mV (altematively: +20-30mV as given In previous samples)

### Example 6:

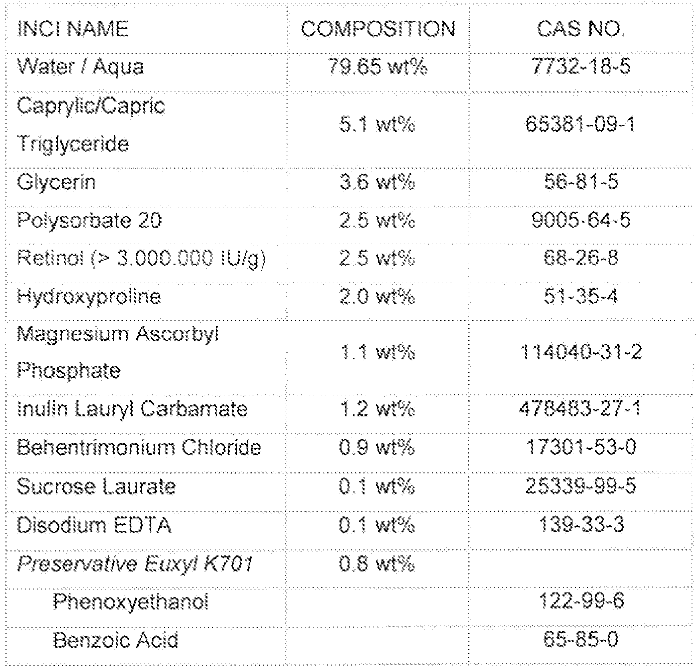

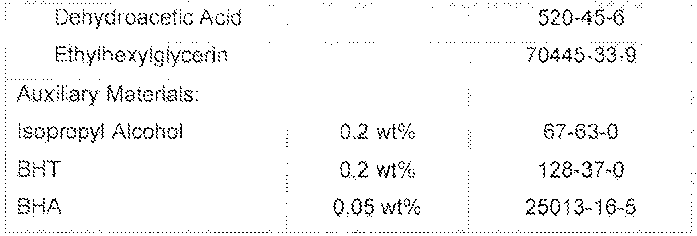

### Manufacturing

Water Phase consisting of water, inulin lauryl carbamate, sucrose laurate, behentrimonium chloride, isopropyl alcohol, glycerin, hydroxyproline and magnesium ascorbyl phosphate were premixed in a stainless steel vessel at a temperature of 60°C, after that cooled to RT. The ingredients of the oil phase constising of retinol, caprylic/capric triglyceride, BHA, BHT and polysorbate 20 were mixed at RT in a stainless steel vessel and added to the water phase white stirring followed by homogenilation prozess with commercially available equipment (homogenization at high pressure homogenization of about 600 bar at RT. The particle size of the physically stable example was measured to be 102.6 nm and the zeta potential was +20-30mV.

### Specification Parameters

Particle Size: Particle size of Delivery System can be from 10-1000 nm, in some cases from 50 - 500 nm.

"Particle Size" or "diameter" refers to the averaged hydrodynamic diameter as measured from diluted aqueous solutions by Photon Correlation Spectroscopy, also referred to as Dynamic Light Scattering. The particle size of the examples of the sugar based delivery systems were measured by Photon Correlation Spectroscopy using a MALVERN Zetasizer Nano SZ90 by means of a 5 mW He-Ne laser (633 nm), with a measuring angle of 90° at a temperature of 25 °C +/- 0.5 °C, according to the procedure published in the MALVERN operating manual.

Zeta Potential: Zetapotential of Delivery System can be from 1-150 mV. Preferred lipid vesicles have a zeta potential from 10 to 100 mV. The term "zeta potential" describes the electric potential of a shear layer of a moving particle in a suspension. Measurement of the zeta potential can occur by moving particles through an applied electric field. The zeta potential can then be calculated from the resulting velocity in the particles.

The zeta potential in conjunction with the present invention is determined by means of laser Doppler electrophoresis. The measurements then occur in strongly diluted aqueous salt solutions, in which case samples being measured are ordinarily present in concentrations of 0.01-0.1 wt % in 1 mM sodium chloride solution. The pH values of the sample solutions lie in the pH value specification range of the corresponding product being measured in which product-specific pH values in the range of about 4.5 to about 8.0. about 4.5 to about 6.5 and in some cases about 5.5 to about 7.5 are generally encountered.
The zeta potential of the examples of the sugar based delivery systems were measured MALVERN Zetasiur Nano SZ90 following the afore-mentioned protocol and procedure according to the instruction manual of the equipment.

### Determination of Topical Epidermal Delivery

For purposes of this invention, the epidermal delivery of active compounds incorporated within inventive compositions was investigated with in vivo 5D-IVT using a multiphoton tomography instrument manufactured by JenLab (JenLab GmbH, Jena, Germany) at ambient temperatures and atmospheric conditions. The inventive composition containing an active compound was non-occlusively applied to the inner forearm. After a period of up to about 5 hours from application of the inventive composition, the treated area was cleaned with a compress that was washed and subsequently dried with a clean, dry compress. After the cleaning procedure the 5D-IVT probe was adjusted to the treated area on the inner forearm of the panelist and the skin was irradiated with near infrared radiation of a wavelength of 700 nm provided by a Titanium:Sapphire tuneable laser system (Mai Tai, Newport Spectra-Physics, USA). The excitation laser beam was attenuated by the use of a Glan calcite polarizer and scanned by two galvanometric mirrors. After passing a beam expander and collimator, the laser pulses are reflected by a dichroic beam splitter into a 40x oil immersion microscope objective with a numerical aperture of 1.3 (Cart Zeiss Jena GmbH, Jena, Germany). The emitted fluorescence light was transmitted and cleared by an additional shortwave-pass filter (F37-490 BrightLine HC 490/LP and F75-680 Muitiphoton-Emitter HC 680/SP, F39-461 BrightLine HC 460/60, F39-390 BrightLine HC 390/40; Semrock Inc., Rochester, NY, USA) and split up in three spectral regions by a set of dichroic beam splitters (F43-031 425DCXR, F33-499 4950CXR: Chroma Technology Corp., Bellows Falls, VT, USA). Three separate photomultipliers (PMTs) provided intensity images of the sample and each PMT was readout by time-correlated single photon counting to calculate wavelength-range-specific fluorescence lifetime values. The data of the PMTs were processed by a high-resolution time-correlated single photon counting imaging module (SPC 830, Becker & Hickl GmbH, Berlin, Germany) in order to monitor and record the in vivo fluorescence image of the human skin. Subsequently the relative dermal penetration of active compounds was determined based on the intensities of the characteristic Fluorescence-lifetime imaging signal. Any of various published modes of operation of 5D-IVT instruments, such as those described in "A comparative study of different instrumental concepts for spectrally and lifetime-resolved multiphoton intravital tomography (5D-IVT) in dermatological applications" (Proceedings of SPIE Vol. 7568), for example, may be suitable to image targeted topical epidermal delivery of active compounds.

In certain embodiments and without wishing to be bound by any theory or explanation, topical epidermal delivery may be established in a 5D-IVT image by a count of discrete domains within the image that are assigned to the active compound. In some cases, the count of discreet domains assigned to the active compound in an image area of 60 µm x 60 µm obtained at a depth of 1 µm to 100 µm is increased.
Most preferable results were obtained by the encapsulation of retinol or Vitamin A given in Example 2c. Determination of stabilization of active ingredients

Active ingredients can be detected by any known analytical test method which can be e.g. HPLC, GC, ESR depending on the composition. This effect depends on the active lipophilic ingredient encapsulated.
Most preferable results were obtained by encapsulation of Retinol or vitamin A given in Example 2. These results illustrate that the instant invention can stabilize Retinol and thereby reduce, if not prevent, Retinol from decomposing or degrading. Accordingly the inventive composition enhances the amount of Retinol that is available for skin penetration (as well as the ability for Retinol to penetrate the skin).

### Test Method used: HPLC Chromatography

### Chromatographic System:

| | |
|---|---|
| Instrumentation: | Liquid Chromatograph Agilent 1100 with autosampler, UV-detector |
| Column: | Reprosil C-18-AQ; 5µ, 200 * 4 mm (Fa. Dr. Maisch) with precolumn |
| Column Temp.: | 20°C |
| Mobile Phase: | Acetonitrile/THF/Water (54/38/8 v/v/v) |
| Flow Rate: | 1.0 ml / min |
| Gradient Program: | Isocratic |
| Detection: | UV-Detector; 323 nm |
| Injection Volume: | 5 µl; Autosampler |
| Retention Time: | 1.6 min |
| Reagents: | |
| Acetonitrile (Roth, gradient grade) | |
| THF (Roth, gradient grade) | |
| 2-Propanol (Roth, gradient grade) | |
| Water, twice-distilled | |
| Retinol 50C (BASF, Cosmetic Quality, approx. 50 %) - standard | |
| (If not stated otherwise the reagents are used as purchased without any further purification) | |

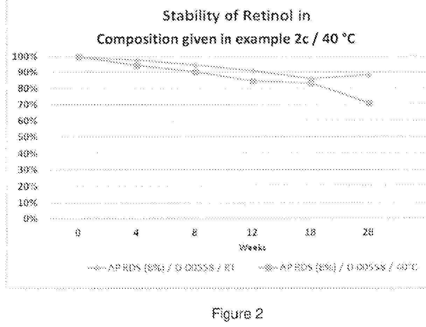

Referring now to Figure 2, Figure 2 shows the results of the HPLC determination of retinol obtained for composition given in example 2c upon storage for 28 weeks.

It must be recognized that in the manufacturing process for the instant invention, the encapsulation efficiency of some actives, such as Retinol, may be less than that achieved by other encapsulation approaches. In particular, it has been observed that despite high Retinol stability described in HPLC studies as above, the invention as described in Example 2 can nonetheless manifest greater than expected color build associated with retinol degradation when exposed to elevated 40°C temperatures in as little as two weeks. This color build can be due to a much larger level of unencapsulated/residual Retinol within the system.

The inventive delivery system imparts improved chemical stability to active ingredients such as retinol. In particular, HPLC analysis confirms that the inventive delivery system reduces chemical degration of retinol whereas unencapsulated retinol degrades.

### Reduction of irritation potential

Reduction of irritation can be detected by any Known analytical test method which can be e.g. 5D IVT, ELISA, Patch Test, HET Cam Test using skin, skin models, cells. This effect depends on the active lipophilic ingredient encapsulated.

Most preferable results were obtained by encapsulation of Retinol or Vitamin A given in Example 2c. The irritation potential of not encapsulated retinol is significantly higher than that of non encapsulated retinol.
Test Method used: 5 D intravitaltomography
Product Application: 0,3 % of retinol occlusively applied onto inner forearm
Measurement of tauₘ: at start point and 48 h after application

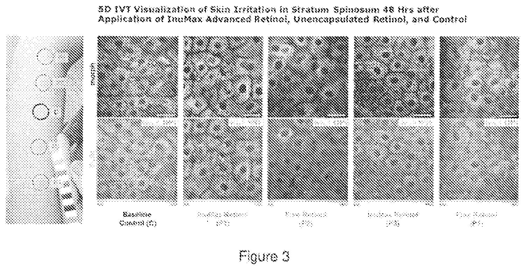

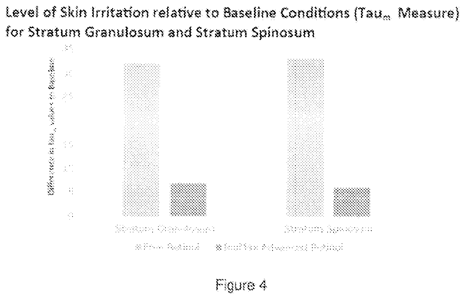

In a repetitive test with example 2c according to a slightly modified test protocol and involving visualization of the irritative potential of retinol we obtained a significant reduction of the retinol irritation potential by encapsulation within the sugar based delivery system of present invention. Data which measured in accordance with the method as described in "From Morphology To Biochemical State - Intravital Multiphoton Fluorescence Lifetime Imaging Of Inflamed Human Skin", by Volker Huck et al., published in www.naturecom/scientificreports, 23 March 2016; the disclosure of which is hereby incorporated by reference. Referring now to Figures 3 and 4, Figures 3 and 4 illustrate the Tauₘ measurement. The delivery system of the invention can achieve Tau values of about 100ps to about 3000ps, about 300ps to about 2500ps and in some cases about 500ps to about 1500ps in the Stratum granulosum and Stratum spinosum.
Personal care products and exemplary formulations using Delivery Systems

The Delivery System compositions disclosed herein may be employed as an ingredient in creating a Cosmetic or Personal Care product. Delivery System compositions may also provide delivery enhancement

and increase the efficacy of other actives, whether the actives are a component of the Delivery System or an ingredient of the Personal Care product. The Delivery System composition is generally present at about 0.1 to about 20% by weight in such products, in some cases between about 0.5 and about 15% by weight, and in still other cases is present between about 1 % and about 10% by weight. The composition can be used with a wide range of personal care products having a base media that can comprise at least one member selected from the group consisting of cosmetic oil (i.e. oils compatible for cosmetic uses), water, alcohol and combinations thereof, all by way of example only.

The Delivery System composition can be added to Personal Care products as is, or as dispersion in water or any suitable organic solvent or combination of solvents and/or water.

Exemplary cosmetic, toiletry and topical health care type personal care products in which the Delivery System composition may be employed include, without limitation, leave-on personal care products (i.e., products that are left on keratinous substrates after application); rinse-off personal care products (i.e., products that are washed or rinsed from keratinous substrates during or within a few minutes of application); at least one of shampoos; chemical and non-chemical hair curling and hair straightening products; hair style maintaining and hair conditioning products; lotions and creams for nails, hands, feet, face. scalp and/or body; hair dye; face and body makeup: nail care products; astringents; deodorants; antiperspirants; anti-acne; antiaging; depilatories; colognes and perfumes; skin protective creams and lotions (such as sunscreens); skin and body cleansers; skin conditioners; skin toners; skin firming compositions; skin tanning and lightening compositions; liquid soaps; bar soaps; bath products; shaving products; and oral hygiene products (such as toothpastes, oral suspensions, and mouth care products), any or the foregoing of which may additionally contain pharmaceutical, ptrytopharmaceutical and/or neutraceutical ingredients.

The form of such Personal Care products includes, without limitation, a liquid, gel, spray, emulsion (such as lotions and creams), shampoo, pomade, foam, tablet, stick (such as lip care products), makeup, suppositories, among others, any of which can be applied to the skin or hair and which typically are designed to remain in contact there with until removed, such as by rinsing with water or washing with shampoo or soap. Other forms could be gets that can be soft, stiff, or squeezable. Emulsions can be oil-in-water, water-in-cil, water-and-silicone, or multiphase. Sprays can be non-pressurized aerosols delivered from manually pumped finger-actuated sprayers or can be pressurized aerosols. In some embodiments, exemplary Delivery System composition are formulated in aerosol compositions such as mousse, spray, or foam forming formulation, where a chemical or gaseous propellant is used.

Personal Care products containing a Delivery System, can be broadly categorized as gel or emulsions products, water-in-oil emulsions, oil-in-water emulsions, or aqueous or alcohol based systems.

*Water-in-oil emulsions* can be prepared by mixing together (1) a heated (i.e., melted) solution of the oil phase and (2) an aqueous phase, the aqueous phase being at a temperature similar to the oil (typically within about 10 °C); and then cooling the mixture while stirring. While bringing the temperature of emulsion down Delivery System composition could be added below 45C as emulsion been emulsified and in process of adding other temperature sensitive cosmetic ingredients like preservative, actives and fragrances. Alternativelyl, the Delivery System composition could be added to the aqueous phase if formulation is made without any heating like cold process formulations. Delivery System composition is generally added below 45 °C to room temperature in any cosmetic formulation. Regardless of the manner in which the Delivery System composition is added, the ratio of the aqueous phase to the oil phase can be, for example, about 0.5:1 to about 9:1.

The amount of the inventive Delivery System employed can varying depending upon a given formulation. The amount of Delivery System can range from about 0.1 to about 4%, about 1 to about 20% and in some cases about 5 to about 14% of a formulation. The following are non-limiting examples of cosmetic formulations comprising water-in-oil emulsions that can include the inventive Composition / Delivery System for active cosmetic compounds:
A) Skin Moisturizer
   Water - about 50 to about 90 wt%
   Silicone - about 1 to about 10 wt%
   Emulsifier - about 0.5 to about 5 wt%
   Emollient - about 5 to about 20 wt%
   Delivery System composition - about 0.1 to about 20 wt%
   Polymer - about 0.1 to about 5 wt%
   Other Additives or Actives - about 0.1 to about 3 wt%
B) Sunscreen
   Water - about 50 to about 90 wt%
   Silicone - about 1 to about 10 wt%
   Emulsifier - about 0.5 to about 5 wt%
   Emollient - about 5 to about 20 wt%
   Delivery System composition - about 0.1 to about 20 wt%
   Polymer - about 0.1 to about 5 wt%
   Sunscreen Active - about 1 to about 25 wt%
   Other Additives or Actives - about 0.1 to about 3 wt%
C) Antiperspirant Deodorant
   Water - about 50 to about 90 wt%
   Silicone - up to about 10 wt%
   Emulsifier - about 0,5 to about 5 wt%
   Ethanol or other solvents - about 0 to about 20 wt%
   Delivery System composition - about 0.1 to about 20 wt%
   Polymer - up to about 5 wt%
   APDO actives - about 0.1 to about 30 wt%
   Other Additives or Actives - about 0.1 to about 5 wt%

*Oil-in-water emulsions* are prepared by mixing together (1) a heated (i.e., melted) solution of the oil phase and (2) an aqueous phase, the aqueous phase being at a temperature similar to the emollient solution (typically within about 10 °C); and then cooling the mixture while stirring. As with the water-in-oil emulsions, the Delivery System composition may initially be added to the aqueous phase if formulation is cold processable or added post-emulsification below 45 °C. The ratio of the oil phase to the water phase can be, for example, about 0.1:1 to about 1;1.

The following are non-limiting examples of cosmetic formulations comprising oil-in-water emulsions:
A) Skin Moisturizer
   Water - about 50 to about 90 wt%
   Emulsifier - about 0.5 to about 5 wt%
   Emollient - about 1 to about 20 wt%
   Delivery System composition - about 0.1 to about 20 wt%
   Polymer - about 0.1 to about 5 wt%
   Other Additives or Actives - about 0.1 to about 3 wt%
B) Sunscreen
   Water - about 50 to about 90 wt%
   Emulsifier - about 0.5 to about 5 wt%
   Emollient - about 1 to about 20 wt%
   Ethanol and/or other organic solvent - up to about 35 wt%
   Delivery System composition - about 0. 1 to about 20 wt%
   Polymer - about 0.1 to about 5 wt%
   Sunscreen Active - about 1 to about 25 wt%
   Other Additives or Actives - about 0.1 to about 3 wt%
C) Mousse or other hair styling product
   Water - about 50 to about 90 wt%
   Emulsifier - about 0.5 to about 1 wt%
   Surfactant - about 0.1 to about 2 wt%
   Delivery System composition - about 0.1 to about 20 wt%
   Polymer - up to about 5 wt%
   Other Additives or Actives - about 0.1 to about 2 wt%
   Solvent - about 1 to about 25 wt%
   Propellent- up to about 10 wt%
D) Antiperspirant Deodorant
   Water - about 50 to about 90 wt%
   Silicone - up to about 10 wt%
   Emulsifier - about 0.5 to about 5 wt%
   Ethanol or other solvents - about 1 to about 35 wt%
   Delivery System composition - about 0.1 to about 20 wt%
      Polymer - up to about 5 wt%
      APDO actives - about 0.1 to about 30 wt%
      Other Additives or Actives - about 0.1 to about 5 wt%

The following are non-limiting examples of cosmetic formulations comprising *alcohol or aqueous systems that can include the inventive compositions.*
A) Coloring Shampoo
   Water - about 50 to about 90 wt %
   Surfactant - about 2 to about 20 wt%
   Foam booster -up to about 20 wt%
   Delivery System composition - about 0.1 to about 20 wt%
   Polymer - up to about 5 wt%
   Other Additives or Actives - about 0.1 to about 10 wt%
B) Hair Spray (aerosol and non-aerosol)
   Water - about 10 to about 90 wt%
   Delivery System composition - about 0.1 to about 20 wt%
   Polymer - up to about 5 wt%
   Ethanol or other solvents - about 10 to about 90 wt%
   Optional Propellant for an aerosol - up to about 50 wt%
   Other Additives or Actives - about 0.1 to about 2 wt%
C) Shampoo
   Water - about 50 to about 90 wt%
   Surfactant - up to about 20 wt%
   Foam booster - about 2 to about 20 wt%
   Detivery System composition - about 0.1 to about 20 wt%
   Polymer - up to about 5 wt%
   Other Additives or Actives - about 0.1 to about 10 wt%
D) Hair Styling products
   Water - about 10 to about 90 wt%
   Delivery System composition - about 0.1 to about 20 wt%
   Polymer - about 0.1 to about 5 wt%
   Ethanol or other solvents- up to about 10 wt%
   Other Additives or Actives - about 0.1 to about 10 wt%
E) Body cleansing products
   Water - about 50 to about 90 wt%
   Surfactant - about 2 to about 20 wt%
   Delivery System composition - about 0.1 to about 20 wt%
   Polymer - up to about 5 wt%
   Foam booster - up to about 20 wt%
   Other Additives or Actives - about 0.1 to about 10 wt%,

In products in which emollients are employed, any suitable emollient for use in cosmetic compositions can be used. Examples of suitable emollients include at least one of esters (e.g., C12-15 alkyl benzoate) and triglycerides (e.g., Caprylic/caprylate triglyceride); hydrocarbon oils (e.g., mineral oil), natural oil (e.g., Jojoba oil, safflower oil), tridecyl trimellitate, sunflower oil, castor oil, among other compounds used to impart desired or improved sensory or aesthetic properties of a personal care composition. Many emollients used in cosmetic compositions also fall within the category of cosmetic oils.

In products in which emulsifiers are employed, any suitable cosmetic emulsifier having a hydrophilic-lipophilic balance (HLB) in the range of about 1 to about 20 can be used. The emulsifier can be nonionic, cationic, anionic, or amphoteric or a combination of such emuisifiers can be used.

Examples of nonionic emulsifiers are at least one of laureths, e.g. laureth-4: ceteths, e.g. ceteths-1; polyethylene glycol cetyl ether; Cetearth, e.g. cetearth-25; polyglycol fatty acid glycerides; hydroxylated lecithin; lactyl esters of fatty acids; and alkyl polyglycosides.

Examples of cationic emulsifiers are at least one of cetyldimethyl-2-hydroxyethylammonium dihydrogenphosphate; cetyltrimonium chloride; cetyltrimonium bromide; cocotrimonium methosulfate; as well as emulsifiers that contain quaternized nitrogen.

Anionic emulsifiers include, for example, at least one of alkyl sulfates; alkyl ether sulfates; alkylsulfonates; alkylarylsulfonates; alkyl succinates; alkyl sulfosuccinates; N-alkylsarcosinates; acyl taurates; acyl isethionates: alkyl phosphates: alkyl ether phosphates; alkyl ether carboxylates; alpha-olefinsulfonates, and the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units,

Surfactants and/or foam boosters may also be employed and like the emulsifiers can be nonionic, cationic, anionic, or amphoteric or a combination of such surfactants can be used.
Suitable anionic surfactants are for example, at least one of alkyl sulfates; alkyl ether sulfates; alkylsulfonates; alkylarylsulfonates; alkyl succinates; N-alkylsarcosinates; acyl taurates; acyl isethionates; alkyl phosphates; alkyl ether phosphates: alkyl ether carboxylates; alpha olefinsulfonates, and may include the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Suitable amphoteric surfactants are, for example, at least one of alkylbetaines; alkylamidopropylbetaines; alkylsulfobetaines; alkyl glycinates; alkylcarboxyglycinates; alkyl amphoacetates or amphopropionates; and alkyl amphodiacetates or amphodipropionates. For example, it is possible to use cocodimethylsulfoprapylbetaine, laurylbetaine, and cocamidopropylbetaine or sodium cocamphopropionate as surfactants,

Examples of nonionic surfactants are the reaction products of aliphatic alcohols having between 6 and 20 carbon atoms in the alkyl chains, which can be linear or branched with ethylene oxide and/or propylene oxide. Also suitable are at least one of alkylamine oxides; mono- or dialkylalkanolamides; fatty acid esters of polyethylene glycols; alkyl polyglycosides and sorbitan ether esters.

Examples of cationic surfactant are quaternary ammonium compounds, for example, cetyltrimethylammonium chloride, as well as other surfactants that contain a quaternized nitrogen.
in personal care compositions in which polymers are used with the Delivery System composition, any one or more polymer(s) can be of any type, including those which are at least one of nonionic, amphoteric or zwitterionic, anionic, cationic or mixtures of such types of polymers.

Exemplary synthetic polymers include at least one of vinyl pyrrolidone or acrylate homopolymer and copolymers, including carbomer, and those which have a vinyl acetate group or acrylate or acrylamide or taurate group. Natural non-ionic polymers suitable for the composition of the present invention can comprise at least one of cellulose, starches, chitosan, xanthan gum, guar gum, neutralized shellac and their derivatives. Cationic derivatives or chemical modified forms of natural polymers may also be included, such as chemically modifies starches, celluloses, guar and xanthan gum e.g., methylcellulose, hydroxyethylcellulose, quaternized guar.

The polymer may also include silicone compounds, such as, for example, at least one of polydiorganosiloxanes, polydialkylsiloxanes, polyalkylsiloxanes, polyarylsiloxane, silicone resins, silicone gums or dimethicone copolyols and aminofunctional silicone compounds such as amodimethicone. Other silicone compounds include graft polymers of organosiloxane and polyethyloxazolines compounds known with the INCI name Polysilicone-9. Any polymeric compound having an INCI name including silicone, methicone, dimethicone, or siloxane as part of its name may be used.

In products in which propellant or solvents are employed, those may include at least one of isobutane, butane, dimethyl ether, and ethanol, among others.

Other Additives or Actives include one or more of additive compounds, active compounds, and moisturizing and humectants compounds. Examples of other additive compounds include one or more members selected from the group consisting of silicone based plasticizers, natural or synthetic compounds (e.g., polysaccharides, natural or synthetic gums, stabilizers, anionic and nonionic associative thickener or theology modifiers soluble in oil or water phase), among other compounds. The additives may include at least one compound selected from the group consisting of preservatives, stabilizers (e.g., Xanthan Gum), antioxidant (e.g., Vitamins), rheology modifiers, fragrances, and pigments, among other additives.
In some personal care products, active compounds that interact with or protect skin or hair can be included. Examples of such active compounds include at least one of sunscreen compounds (e.g. zinc oxide, titanium dioxide, octinoxate, octocrylene, ethylhexyl salicylate, oxybenzone); skin whiteners (e.g. salicylic acid, aloesin, ethyl ascorbic acid, arbutin); anti-cellulite compounds; anti-aging compounds (e.g., polypeptides such as Argininie/Lysine, Argininie PCA, Aspergillus/Aspidosperma Quebracho Ferment, botanical actives, Bifida Ferment Lysate, Calophylum Inorhylum seed oil, camellia sinensis extract, ceramides, chlorella vulgaris extract, coriolus versicolor extract, corylus avelfana (hazel) seed extract, erythorbic acid, hydrolyzed elastins, hydrolyzed proteins, hydrolyzed soy flour, hydrolyzed peptides, and Vitamins A, E, C, K, B7, and B5 as well as Niacinamide); anti-acne compounds (e.g., salicylic acid, sodium salicylate, benzoyl peroxide); anti-dandruff compounds (e.g., zinc pyrithione); APDO compounds (e.g., aluminum chlorohydrate, aluminum zirconium tetra chlorohydrex); vitamins (e.g., Tocopherol natural, synthetic Tocopherol, synthetic tocopheryl acetate, Retinol, Retinyl palmitate, Retinyl acetate, Provitamin B-5, ascorbic acid, sodium ascorbyl phosphate, Ascorbyl glucoside, Magnesium ascorbyl phosphate); insect repellents; and Coenzymes and Enzymes (e.g., Ubiquinone, Coenzyme Q10); Botanicals (e.g., Aloe vera, Green tea extract, Grape seed extract, Isoflavones, Chamomille/bisabolol, Fennel, Ginko, Ginseng, Guava): Alpha Hydroxy Acids (e.g., Citric acid, Glycolic acid, Lactic acid); Sugar cane extracts; Avena Sativa (Oat) Kernel Protein, and Avocado Sterols, proteins, peptides, copper peptides, fermented biopolymers, beta-glucan, caffeine. Hyaluronic acid and its derivatives like sodium hyaluronate, all by way of example only, It will be appreciated that certain active compounds may fall into more than one category and/or be used to accomplish more than one result.

Active compounds may also include at least one in vivo Fluorescent Dye. While an in vivo Fluorescent Dye may not interact with or protect skin or hair, it may be used as a model for cosmetic actives to allow for the assessment and prediction of epidermal penetration of cosmetic actives, in vivo Fluorescent Dyes include any fluorescent dye that meets regulatory and/or safety requirements for human in vivo skin penetration studies, and includes Fluorescein Alcon 10%, for example.

In Personal Care products, moisturizing and humectants compounds that interact with or skin or hair could be simple or complex mixtures of chemical agents specially designed to make the external or upper layers of the skin (epidermis) softer and more pliable. They increase the skin's hydration or water content by reducing evaporation or by bringing moisture to the skin. Types of moisturizers and Humectants include, for example, naturally occurring skin lipids and sterols, as well as artificial or natural oils, emollients, lubricants. Examples of such moisturizing or humectants compounds include glycol (e.g., glycerin, propylene glycol, dipropylene glycol, butylene glycol MP diol, sorbitol, and hexylene glycol), Polysaccharides (e.g., Hyaluronic acid and its salts, B-1, 3-glucans, Chitosan), Botanicals (e.g., Aloe vera, Ginkgo, green tea extract, rose extract, sugar extract). Natural oils (e.g., coconut oil, grape seed oil, jojoba oil, olive oil, Argan oil), polyethylene glycol ether of methyl glucose (e.g., Methyl Gluceth-10), green algae, natural and herbal extract design for moisturization like alpha lipoic acid, peptides, proteins amino acids and collagens and betaine among others.

### Examples Product Embodiment of Exemplary Formulations using Delivery System

### Example Product 1. Skin Cream Containing Inventive Sugar based Delivery System

| Ingredient | % weight |
|---|---|
| Sugar based Delivery System | 3.00 |

| Phase A | |
|---|---|
| Deionized Water | Balance |
| Glycerol | 5.00 |
| Xanthan Gum | 0,65 |

| Phase B | |
|---|---|
| Dicaprylyl. Ether | 5.00 |
| Octyldodecanol | 3.00 |
| Isomerized Safflower Acid (Clarinol® A-80) | 3.00 |
| Arachidyl Alcohol, Behenyl Alcohol, Arachidyl Glucoside (Montanov™ 202) | 3.00 |

| Phase C | |
|---|---|
| Glyceryl Caprylate | 1.00 |

The ingredients of phase A were added together, mixed and heated to 75 °C. The ingredients of phase B were added to a separate container, heated to 75 °C until melted, and mixed. The phase B mixture was added to phase A while mixing, followed by homogenisation. The mixture was cooled to 60 °C and phase C was adding with gentle mixing. The mixture was further homogenised for a short time, cooled to 40 °C, and the Sugar based delivery system was added with gentle mixing until homogenous,

### Example Product 2. Second Skin Cream Containing Inventive Sugar based Delivery System

A Skin Cream is prepared according to the composition and procedure of Example Product 1, with the Sugar based delivery system at 5 wt%.

### Example Product 3. Second Skin Cream Containing Inventive Sugar based Delivery System

| Ingredient | % weight |
|---|---|
| Phase A | |
| Deionized Water | balance |
| Glycerol | 3.0 |

| Phase B | |
|---|---|
| Sodium Behenoyl Lactylate | 2.0 |
| Cetearyl Alcohol | 3.0 |
| Glyceryl Stearate | 2.6 |
| Isopropyl Palmitate | 6.0 |
| Simmondsia Chinensis Seed Oil | 6.0 |

| Phase C | |
|---|---|
| Sugar based Delivery System | 10.0 |
| Preservative of Phenoxyethanol (71-77 wt%), Benzoic Acid (11-13 wt%), Dehydroxyacetic Acid (6.5-7.5 wt%), Ethylhexylglycerin (1.4- 11.4 wt%), Polyaminopropyl Biguanide (0.1- 1,1 wt%) (Euxyl® K702 from Schülke & Mayr GmbH] | 0.5 |

The ingredients of phase A were added together, mixed and heated to 80 °C. The ingredients of phase 8 were added to a separate container, mixed and heated to 80 °C. The phase B mixture was added to phase A while mixing and homogenizing. The mixture was cooled to 40 °C and ingredients of phase C were added with gentle mixing until the cream was homogeneous.

### Example Product 4. Third Skin Cream Containing Inventive Sugar based Delivery System

A Skin Cream is prepared according to the composition and procedure of Example Product 3, with the Sugar based delivery system at 5 wt%

### Example Product 5. Fourth Skin Cream Containing Inventive Sugar based Delivery System

| Ingredient | % weight |
|---|---|
| Phase A | |
| Deionized Water | balance |
| Glycerol | 3.0 |
| Betaine | 1.5 |
| Xanthan Gum | 0.2 |

| Phase B | |
|---|---|
| Polyglyceryl-3 Methylglucose Distearate | 3.8 |
| Cetearyl Alcohol | 5.4 |
| Simmondsia Chinensis Seed Oil | 2.9 |
| Persea Gratissima Seed Oil | 4.2 |
| Mangifera indica Seed Butter | 3.5 |

| Phase C | |
|---|---|
| Sugar based Delivery System | 10.0 |
| Preservative of Phenoxyethanol (28.3-34.3 wt%), Benzyl Alcohol (28.3-34.3 wt%), Potassium Sorbate (17.0-20.6 wt%) [Euxyl® K700 from Schülke & Mayr GmbH] | 1.0 |

The Skin Cream was prepared according to the procedure of Example Product 3.

### Example Product 6. Fifth Skin Cream Containing Inventive Sugar based Delivery System

A Skin Cream is prepared according to the composition and procedure of Example Product 5, except that the Sugar based delivery system is used at 5 wt%.

### Example Product 7. Sixth Skin Cream Containing Inventive Sugar based Delivery System

| Ingredient | % weight |
|---|---|
| Phase A | |
| Deionized Water | balance |
| Triticum Vulgare Kernel Flour | 6.0 |

| Phase B | |
|---|---|
| Glyceryl Stearate Citrate | 4.3 |
| Cetearyl Alcohol | 3.6 |
| Decyl Cocoate | 3.0 |
| Simmondsia Chinensis Seed Oil | 5.2 |
| Dicaprylyl Carbonate | 2.3 |
| Dicaprylyl Ether | 1.7 |

| Phase C | |
|---|---|
| Sugar based Delivery System | 10.0 |
| Preservative of Benzyl Alcohol (79 wt%), Benzoic Acid (12 wt%), Dehydroacetic (8 wt%) | 1.0 |

The Skin Cream was prepared according to the procedure of Example Product 3.

### Example Product 8. Seventh Skin Cream Containing Inventive Sugar based Delivery System

A Skin Cream is prepared according to the composition and procedure of Example Product 7, except that the Sugar based delivery system is used at 5 wt%.

### Example Product 9. Eighth Skin Cream Containing Inventive Sugar based Delivery System

| Ingredient | % weight |
|---|---|
| Phase A | |
| Deionized Water | balance |
| Triticum Vulgare Kernel Flour | 6.0 |

| Phase B | |
|---|---|
| Brassica Campestris Seed Oil | 6.1 |
| Simmondsia Chinensis Seed Oil | 7.4 |
| Caprylc/Capric Triglyceride | 4.1 |
| Cetearyl Alcohol | 3.8 |
| Dicaprylyl Carbonate | 2.9 |
| Coco-Caprylate | 1.7 |

| Phase C | |
|---|---|
| Sugar based Delivery System | 10.0 |
| Preservative of Benzyl Alcohol (79 wt%), Benzoic Acid (12 wt%). Dehydroacetic (8 wt%) | 1.0 |

The Skin Cream was prepared according to the procedure of Example Product 3.

### Example Product 10. Ninth Skin Cream Containing Inventive Sugar based Delivery System

A Skin Cream is prepared according to the composition and procedure of Example Product 9, except that the Sugar based delivery system is used at 5 wt%.

### Example Product 11. First Serum Containing Inventive Sugar based Delivery System

| Ingredient | % weight |
|---|---|
| Phase A | |
| Deionized Water | balance |
| Carbomer | 1.3 |

| Phase B | |
|---|---|
| Sodium Hydroxide, 10 wt% in Water | 3.6 |

| Phase C | |
|---|---|
| Sorbeth-30 | 5.0 |
| Preservative of Phenoxyethanol (71-77 wt%), Benzoic Acid (11-13 wt%), Dehydroxyacetic Acid (6.5-7.5 wt%), Ethylhexylglycerin (1,4-11.4 wt%). Polyaminopropyl Biguanide (0.1-1.1 wt%) | 0.5 |
| Sugar based Delivery System | 10.0 |

The ingredients of phase A were added together and mixed at room temperature until the Carbomer was fully dissolved. The ingredient of phase B was added to the mixture for neutralization, Ingredients of phase C were then added in the order given with gentle mixing until the serum was homogeneous.

### Example Product 12. Second Serum Containing inventive Sugar based Delivery System

A Serum is prepared according to the composition and procedure of Example Product 11, except that the Sugar based delivery system is used at 5 wt%.

### Example Product 13. Tenth Skin Cream Containing inventive Sugar based Delivery System

| Ingredient | % weight |
|---|---|
| Phase A | |
| Deionized Water | balance |
| Xanthan Gum | 0.9 |

| Phase B | |
|---|---|
| Isopropyl Myristate | 10.0 |
| Jojoba Oil | 1.0 |
| Cyclopentasiloxane | 5.0 |
| Cetearyl Alcohol (and) Dicetyl Phosphate (and) Ceteth-10 Phosphate | 6.0 |
| Bis-PEG/PPG-14/14 Dimethicone | 2.5 |
| Apricot Kernel Oil | 0.4 |
| Sweet Almond Oil | 0.6 |

| Phase C | |
|---|---|
| Sugar based Delivery System | 3,0 |
| Propylene glycol, Diazolidinyl urea, Methylparaben. Propylparaben | 1.0 |
| Triethanolamine | as needed to achieve final pH 5.7 |

The ingredients of phase A were added together, mixed and heated to 85 °C The ingredients of phase B were added to a separate container, mixed and heated to 85 °C. The phase B mixture was added to phase A and mixed for 3 minutes, cooled to 65 °C, and homogenized at 6000 rpm for 3 minutes. The mixture was cooled with stirring to between 40 °C and 20 °C, and ingredients of phase C were then added in the order given with gentle mixing until the cream was homogeneous.

### Example Product 14. Eleventh Skin Cream Containing inventive Sugar based Delivery System

A Skin Cream is prepared according to the composition and procedure of Example Product 13, except that the Sugar based delivery system is used at 5 wt%.

### Example Product 15. Twelfth Skin Cream Containing Inventive Sugar based Delivery System

| Ingredient | % weight |
|---|---|
| Phase A | |
| Deionized Water | balance |
| Xanthan Gum | 0.9 |

| Phase B | |
|---|---|
| Mineral Oil | 12.5 |
| PEG-30 dipolyhydroxy stearate | 1.0 |
| Steareth-2 | 2.0 |
| Steareth 21 | 1.0 |
| Dimethicone | 4,5 |
| C12-15 Alkyl Benzoate | 4.5 |

| Phase C | |
|---|---|
| Sugar based Delivery System | 3.0 |
| DMDM Hydantoin | 0.5 |

The Skin Cream was prepared according to the procedure of Example Product 13.

### Example Product 16. Thirteenth Skin Cream Containing Inventive Sugar based Delivery System

A Skin Cream is prepared according to the composition and procedure of Example Product 15, except that the Sugar based delivery system is used at 5 wt%.

### Example Product 17. Fourteenth Skin Cream Containing inventive Sugar based Delivery System

| Ingredient | % weight |
|---|---|
| Phase A | |
| Deionized Water | balance |
| Xanthan Gum | 0.2 |

| Phase B | |
|---|---|
| Octinoxate | 1.0 |
| Cetearyl Alcohol | 6.0 |
| Ceteareth-25 | 0.5 |

| Phase C | |
|---|---|
| Sugar based Delivery System | 3.0 |
| Propylene Glycol, Diazolidinyl Urea, Methyparaben, Propylparaben | 1.0 |

The Skin Cream was prepared according to the procedure of *Example* Product 13.

### Example Product 18, Fifteenth Skin Cream Containing inventive Sugar based Delivery System

A Skin Cream is prepared according to the composition and procedure of Example Product 17, except that the Sugar based delivery system is used at 5 wt%.

### Example Product 19. Sunscreen (SPF 15) Containing inventive Sugar based Delivery System

| Ingredient | % weight |
|---|---|
| Phase A | |
| Deionized Water | balance |
| Acrylates/C10-30 Alkyl Acrylate Crosspolymer | 0.3 |
| Acrylate polymer | 1.0 |
| Triethanolamine | 1.0 |

| Phase B | |
|---|---|
| Octocrylene | 3.0 |
| Octisalate | 3.0 |
| Oxybenzone | 2.0 |
| Avobenzone | 1.0 |
| Stearic Acid | 2.0 |
| Glyceryl Monostearate SE | 3.0 |
| Dimethicone | 0.5 |
| C12-15 Alkyl Benzoate | 8.0 |

| Phase C | |
|---|---|
| Sugar based Delivery System | 5.0 |
| Phenoxyethanol & Ethylhexylglycerin | 1.0 |

The Sunscreen is prepared according to the procedure of Example Product 13.

### Example Product 20. Hair Styling and Moisturizing Cream Containing Inventive Sugar based Delivery System

| Ingredient | % weight |
|---|---|
| Phase A | |
| Deionized Water | balance |
| Carbomer | 0.5 |
| Triethanolamine | 0.8 |
| Polyurethane-2 and Polymethyl Methacrylate | 5.0 |
| Polyquatemium-55 | 1.0 |

| Phase B | |
|---|---|
| Ceteareth-20 | 0.5 |
| Polysilicone-11 & Water & Loureth-12 & Phenoxyethanol & Ethylhexyglycerin | 4.0 |
| Dimethicone 100 Cst | 0.1 |
| PEG-12 Dimethicone | 5.0 |
| Jojoba oil | 2.0 |

| Phase C | |
|---|---|
| Sugar based Delivery System | 5.0 |
| Phenoxyethanol & Ethylhexylglycerin | 1.0 |

The Hair Cream is prepared according to the procedure of Example Product 13.

### Example Product 21. Moisturizing BB Cream (Foundation) Containing Sugar Based Delivery System

| | |
|---|---|
| Ingredient | % weight |

| Phase A | |
|---|---|
| Polysilicone-11 & Water & Laureth-12 & Phenoxyethanol & Ethylhexylglycerin | 5.0 |
| Dimethicone 50 Cst | 20,0 |
| PEG-10 Dimethicone | 2.0 |
| Dimethicone & PEG/PPG-18/18 Dimethicone | 2.0 |
| Disteardimonium Hectorite | 0.6 |

| Phase B | |
|---|---|
| Yellow Iron Oxide | 1.3 |
| Red Iron Oxide | 0,4 |
| Black Iron Oxide | 0.1 |
| Titanium Dioxide & Methicone | 6.6 |
| Mica | 6.6 |
| Dimethicone 50 Cst | 7.5 |
| PEG-10 Dimethicone | 2.0 |

| Phase C | |
|---|---|
| Deionized Water | balance |
| Glycerin | 5.0 |
| Butylene Glycol | 3.0 |
| Sodium Chloride | 1.0 |

| Phase D | |
|---|---|
| Sugar based Delivery System | 3.0 |
| Phenoxyethanol & Ethylhexyiglycerin | 1.0 |

The ingredients of phase A are added together, are mixed and heated to 85 °C. The ingredients of phase B are added to a separate container, are mixed and heated to 85 °C. The phase B mixture is added to phase A and is mixed for 3 minutes; the mixture is cooled to 65 °C, and is homogenized at 6000 rpm for 3 minutes. Phase C ingredients are then added to the mixture and mixing is continued until homogeneous. The mixture is cooled with stirring to between 40 C and 20 °C, and ingredients of phase D are then added with gentle mixing until the cream is homogeneous.

### Example Product 22. Hair Conditioner with Moisturization Containing Inventive Sugar Based Delivery System

| Ingredient | % weight |
|---|---|
| Phase A | |
| Deionized Water | balance |
| Polyquaternium-10 | 0.5 |
| Polyquaternium-4 | 0.7 |

| Phase B | |
|---|---|
| PEG 12 Dimethicone | 2.0 |
| Cyclopentasiloxane (and) Cetearyl Dimethicone/Vinyl Dimethicone Crosspolymer | 4.5 |
| Sugar based Delivery System | 3.0 |
| Phenoxyethanol & Ethylhexylglycerin | 1.0 |

The ingredients of phase A are added together and mixed until homogeneous. The ingredient of phase B are added together in a separate container and mixed until homogeneous. Phase B is added to phase A and mixed until the conditioner is homogeneous.

### Example Product 23. Luxuriously Deep Retinol Cream with Sugar-Based Delivery System and Intelimer® 13 Series Polymers (Formulaiton No. S068)

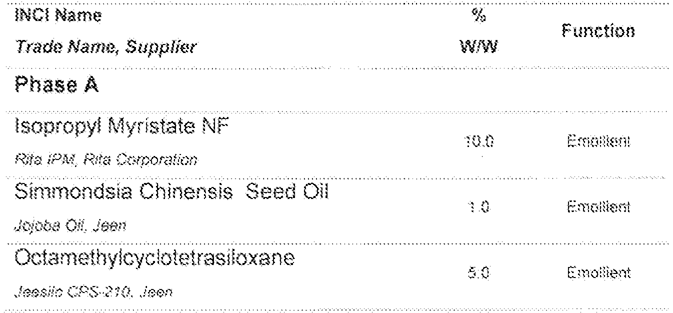

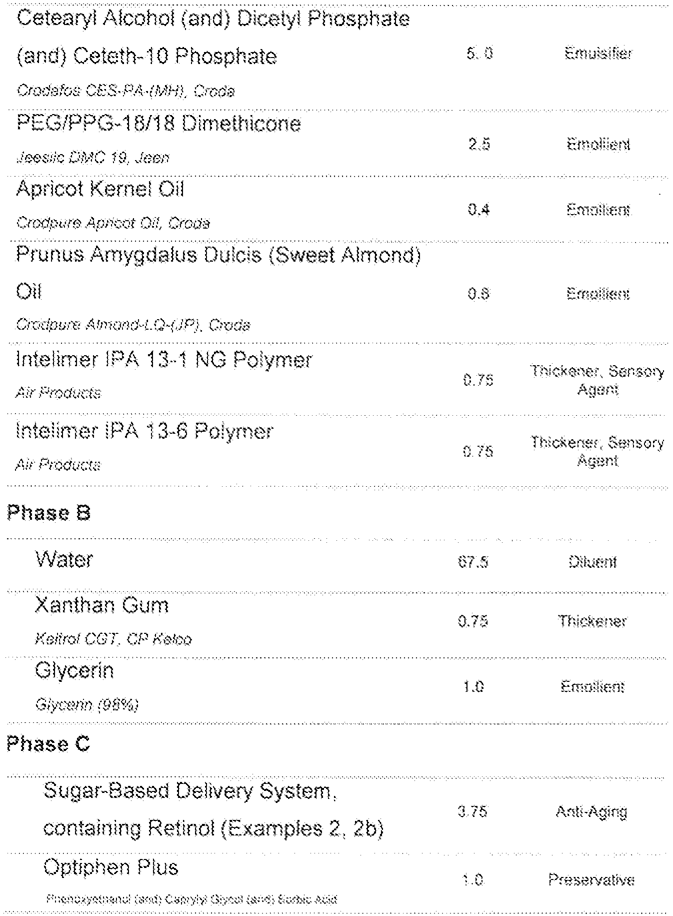

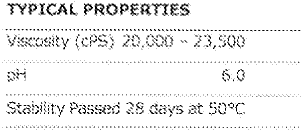

The ingredients of phase A are added together in a vessel. These ingredients are then mixed while heated to 80°C. In a separate vessel, Phase B water is added and begun to be mixed. Xanthan gum is then sifted into this water, while allowing the xanthan gum to fully disperse. Glycerin is then added to this, with continued mixing. The Phase B vessel is then heated to 80°C with continued mixing. Once 80°C temperature has been reached, Phase A was added into Phase B. The mixture was then mixed for 3 minutes and cooled, with mixing, to 65°C. This mixture was then homogenized for 3 minutes at 6000 rpm. The homogenizer was then removed and the mixture was mixed until it has cooled to room temperature. The Sugar-Based Delivery System and preservative system was then added to the mixture, with light mixing. If necessary, the pH of the formulation was then adjusted through the addition of 30% Sodium Hydroxide solution. The formulation can then be packaged.

## Claims

1. Skin penetrating water based composition for cosmetic applications comprising positively charged sugar based vesicles made of
sugar based emulsiflers,
at least one coemulsifier,
positively charged molecules,
solvents selected from water and water soluble solvents,
retinol as lipophilic active,
optional auxiliiary materials like chelating agents and preservatives having a particle size from 50 to 1000 nm and
a zeta potential from 1 to 150 mV,
wherein the emulsifier comprises an inulin backbone modified by linear C8-C22 alkyl groups and
wherein the polyoxyethylene based coemulsifier comprises a polysorbate molecule.

2. The composition of claim 1 further comprising a coemulsifier having a hydrophilic-lipophilic balance (HLB) in the range of about 1 to about 20 and being nonionic, cationic, anionic, or amphoteric or a combination thereof in nature.

3. A Personal Care product comprising the composition of Claim 1.

4. A Cosmetic product comprising the composition of Claim 1.

## Patentansprüche

1. Haut durchdringende Zusammensetzung auf Wasserbasis für kosmetische Anwendungen, umfassend positiv geladene Vesikel auf Zuckerbasis, hergestellt aus Emulgatoren auf Zuckerbasis,
wenigstens einem Coemulgator,
positiv geladenen Molekülen,
Lösungsmitteln, ausgewählt aus Wasser und wasserlöslichen Lösungsmitteln,
Retinol als lipophilem Wirkstoff,
optionalen Hilfsmaterialien wie Chelatbildnern und Konservierungsstoffen,
mit einer Teilchengröße von 50 bis 1000 nm und einem Zeta-Potential von 1 bis 150 mV,
wobei der Emulgator ein Inulingrundgerüst umfasst, das durch geradkettige C8-C22-Alkylgruppen modifiziert ist, und
wobei der Coemulgator auf Polyoxyethylenbasis ein Polysorbatmolekül umfasst.

2. Zusammensetzung nach Anspruch 1, ferner umfassend einen Coemulgator, der einen HLB(Hydrophilic-Lipophilic Balance)-Wert im Bereich von etwa 1 bis etwa 20 aufweist und dessen Eigenschaft nichtionisch, kationisch, anionisch oder amphoter oder eine Kombination davon ist.

3. Körperpflegeprodukt, umfassend die Zusammensetzung nach Anspruch 1.

4. Kosmetikprodukt, umfassend die Zusammensetzung nach Anspruch 1.

## Revendications

1. Composition à base d'eau pénétrant la peau pour des applications cosmétiques comprenant des vésicules à base de sucre chargées positivement composées d'émulsifiants à base de sucre,
d'au moins un coémulsifiant,
de molécules chargées positivement,
de solvants choisis parmi l'eau et des solvants solubles dans l'eau,
de rétinol en tant qu'agent actif lipophile, éventuellement de matériaux auxiliaires comme des agents chélatants et des conservateurs
possédant une taille de particule de 50 à 1 000 nm et un potentiel zêta de 1 à 150 mV,
l'émulsifiant comprenant un squelette d'inuline modifié par des groupes alkyle linéaires en C8 à C22 et,
le coémulsifiant à base de polyoxyéthylène comprenant une molécule de polysorbate.

2. Composition selon la revendication 1 comprenant en outre un coémulsifiant possédant un équilibre hydrophile-lipophile (HLB) dans la plage d'environ 1 à environ 20 et qui est non ionique, cationique, anionique, ou amphotère ou une combinaison de ceux-ci de nature.

3. Produit de soin personnel comprenant la composition selon la revendication 1.

4. Produit cosmétique comprenant la composition selon la revendication 1.
